(19) Europäisches Patentamt
European Patent Office
Office européen des brevets

(11) Veröffentlichungsnummer: **0 045 279**
**B1**

(12) # EUROPÄISCHE PATENTSCHRIFT

(45) Veröffentlichungstag der Patentschrift: **01.06.88**

(21) Anmeldenummer: **81810294.9**

(22) Anmeldetag: **20.07.81**

(51) Int. Cl.[4]: **C 07 C 135/02,** C 07 C 143/78, C 07 D 231/06, C 07 D 249/24, C 07 D 251/16, D 06 L 3/12, C 11 D 3/42

(54) **Neue Aminoxidverbindungen, Verfahren zu deren Herstellung und deren Verwendung als optische Aufheller.**

(30) Priorität: **25.07.80 CH 5708/80**

(43) Veröffentlichungstag der Anmeldung: **03.02.82 Patentblatt 82/5**

(45) Bekanntmachung des Hinweises auf die Patenterteilung: **01.06.88 Patentblatt 88/22**

(84) Benannte Vertragsstaaten: **CH DE FR GB IT LI**

(56) Entgegenhaltungen:
**AT - B - 333 401**
**DE - A - 1 518 638**
**DE - A - 1 643 228**
**DE - A - 2 525 682**
**DE - A - 2 539 461**
**DE - B - 1 291 749**

(73) Patentinhaber: **CIBA-GEIGY AG, Klybeckstrasse 141, CH-4002 Basel (CH)**

(72) Erfinder: **Guglielmetti, Leonardo, Dr., Wartenbergstrasse 6, CH-4103 Bottmingen (CH)**

(74) Vertreter: **Zumstein, Fritz, Dr. et al, Dr. F. Zumstein sen Dr. E. Assman Dipl.-Ing. F. Klingseisen Dr. F. Zumstein jun. Bräuhausstrasse 4, D-8000 München 2 (DE)**

EP 0 045 279 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft neue Aminoxidverbindungen, Verfahren zu deren Herstellung, deren Verwendung als optische Aufheller, vorzugsweise in kationische Textilbehandlungsmittel enthaltenden Waschmitteln, sowie die neuen Verbindungen enthaltende Wasch-, Textilbehandlungs- und Wäschenachbehandlungsmittel.

Bei bisher bekannten fluoreszierenden Verbindungen, die eine N-Oxidgruppe enthalten, handelt es sich immer um Derivate des v-Triazol-N-oxids. Siehe z.B. GB-PSen 1 190 511 und 1 190 514, DE-OS 1 906 662. Meistens sind diese v-Triazol-N-oxid-Verbindungen nur als Zwischenprodukte für die Herstellung von optischen Aufhellern mit einer v-Triazolgruppe beschrieben, z.B. in GB-PSen 1 190 512 und 1 190 514, US-PS 3 819 645, CH-PSen 551 987, 561 707 und 561 709 und DE-OS 2 712 408, wobei die N-Oxid-Gruppe durch Reduktion entfernt werden muss.

Die bekannten N-Oxide von v-Triazolylaufhellern haben den Nachteil, dass sich die stark semipolare N-Oxid-Bindung direkt im konjugierten Aufhellersystem befindet. Dies hat eine teilweise Aufhebung der Konjugation des Systems zur Folge, wodurch auch eine starke Verminderung der Fluoreszenz eintritt. Sie sind daher als optische Aufheller nicht brauchbar und können auch nicht als solche bezeichnet werden. Einige der oben genannten Literaturstellen geben als Verwendungszweck für die genannten v-Triazol-N-oxide die Anwendung als Textilmarkierungsmittel an. Aus der Literatur sind die verschiedensten Typen von optischen Aufhellern bekannt. Beispiele dafür sind in den DE-OSen 2 525 682 und 2 539 461. N-Oxide, die als oberflächenaktive Substanzen beschrieben sind, sind ebenfalls in grosser Zahl bekannt. Siehe z. B. DE-OS 1 643 228, DE-AS 1 291 749 und AT-PS 333 401.

Aufgabe der vorliegenden Erfindung war es, neue optische Aufheller zu schaffen, die einerseits die Vorteile der stark semipolaren N-Oxid-Bindung haben (hohes Dipolmoment und damit Zwitterionencharakter der Verbindungen), ohne die Nachteile der oben beschriebenen bekannten N-Oxidaufheller zu besitzen, und die über hervorragende applikatorische Eigenschaften verfügen.

Insbesondere war es auch Ziel der Erfindung, optische Aufheller zu schaffen, die in Waschmitteln nicht nur mit anionischen Detergentien, sondern auch mit kationischen Tensiden, Textilweichmachern und anderen kationischen Textilhilfsmitteln vor allem im sauren Bereich verträglich sind. In sauren Lösungen bilden die erfindungsgemässen Aminoxide quaternäre Ammonium-Ionen und haben somit auch kationische Eigenschaften.

Gegenüber bekannten kationischen Aufhellern, z.B. solchen, die eine quaternäre Ammoniumgruppe enthalten, haben die erfindungsgemässen Aminoxid-Aufheller den Vorteil, dass sie in neutralem und alkalischem Medium auch als nichtionogene Aufheller fungieren können (Bildung der nicht-ionogenen Hydrate) und sie somit ein breiteres Anwendungsspektrum besitzen. Sie können also auch zusammen mit anionischen Detergentien in üblichen Waschmitteln eingesetzt werden. Ausserdem weisen sie eine höhere Schmutzbeständigkeit, vor allem gegenüber Sebum-Schmutz (Schmutz, der durch Hautabsonderungen entsteht), als bekannte in kationische Textilbehandlungsmittel enthaltenden Waschmitteln verwendbare Aufheller auf.

Die vorstehend beschriebenen Aufgaben konnten überraschenderweise durch die erfindungsgemässen Aufheller gelöst werden, die eine vom konjugierten System des Aufhellers getrennte Aminoxidgruppierung in einem oder zwei Substituenten aufweisen.

Die neuen, erfindungsgemässen Aufheller sind Aminoxidverbindungen der Formel

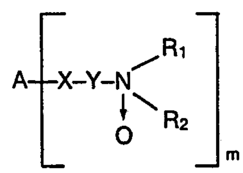,

worin

m die Zahl 1 oder 2,

A den Rest eines Aufhellersystems aus der Gruppe der 2-Furanylbenzimidazole, 2-Azolylbenzimidazole, 2-Stilbenyl-benzoxazole, 2-Stilbenyl-benzimidazole, 1,2-Bis-azolyläthylene, 2,5-Bis-benzimidazolylfurane, 4,4′-Bis-azolylstilbene, 2-Phenyl-5-azolylthiophene, 1,3-Diphenylpyrazoline, 4,4′-Distyrylbiphenyle, 4,4′-Divinyl-stilbene, 1,4-Distyrylbenzole, 3,7-disubstituierten Cumarine, Naphthalimide, 2-Stilben-4-yl-naphthotriazole, 4,4′-Bis-triazolyl- oder -pyrazolyl-stilbene, Triazolyl- oder Pyrazolyl-stilbene, Naphthotriazolylstilbene, Triazinylpyrene oder Bis-styryl-dibenzofurane, wobei diese Reste unsubstituiert oder durch Halogen, Alkyl, Hydroxyalkyl, Halogenalkyl, Cyanoalkyl, Alkoxyalkyl, Phenylalkyl, Carboxyalkyl, Carbalkoxyalkyl, Alkenyl, Cycloalkyl, Alkoxy, Alkenyloxy, Alkoxycarbonyl, Aminocarbonyl, Alkyl- oder Dialkylaminocarbonyl, Cyano, Alkylsulfonyl, Alkoxysulfonyl, Aminosulfonyl, Hydroxy, Carboxy, Sulfo oder Trifluormethyl substituiert sind,

X eine direkte Bindung zwischen A und Y, ein Sauerstoff- oder Schwefelatom oder eine Gruppe der Formel $-SO_2-$, $-SO_2-O-$, $-COO-$, $-CON(R)-$ oder $-SO_2N(R)-$, worin R für Wasserstoff oder unsubstituiertes oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituiertes Alkyl steht,

Y eine unsubstituierte oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituierte geradkettige oder verzweigte Alkylen- oder Alkylenoxyalkylengruppe,

$R_1$ und $R_2$ unabhängig voneinander Cyclohexyl, unsubstituiertes oder durch Halogen, Hydroxy, $C_2$–$C_5$-Carbalkoxy, $C_1$–$C_4$-Alkoxy, Phenyl, Chlor-

phenyl, Methylphenyl, Methoxyphenyl, Carbamoyl oder Sulfamoyl substituiertes Alkyl, Phenyl oder $R_1$ und $R_2$ zusammen mit dem Stickstoffatom, an das sie gebunden sind, einen 5- oder 6-gliedrigen gesättigten oder ungesättigten Heterocyclus, der gegebenenfalls noch ein oder zwei weitere Heteroatome als Ringglieder enthalten kann, wobei gegebenenfalls ein gesättigter Heterocyclus durch 1 oder 2 $C_1$–$C_4$-Alkylgruppen und ein ungesättigter Heterocyclus durch Halogen, Alkyl oder Alkoxy substituiert sein kann, bedeuten; oder worin für den Fall, dass X für eine Gruppe der Formel –CON(R)– oder $-SO_2N(R)-$ und Y für Äthylen oder Propylen stehen, R und $R_1$ gemeinsam die Äthylen- oder Methylengruppe bedeuten, wodurch ein entsprechender, 2 Stickstoffatome enthaltender, gesättigter Heterocyclus entsteht.

In den möglichen Substituenten des Aufhellersystems A haben Alkyl- und Alkoxygruppen, auch in zusammengesetzten Gruppen wie z. B. Alkoxyalkyl, jeweils einzeln z. B. 1 bis 10, vorzugsweise 1 bis 6, insbesondere 1 bis 4 C-Atome. Cycloalkyl hat vorzugsweise 5 oder 6 C-Atome, Alkenyl insbesondere 3 oder 4 C-Atome. Von den Halogenatomen sind Fluor, Chlor und Brom, insbesondere Chlor bevorzugt. Die Zahl der möglichen derartigen Substituenten hängt von der Grundstruktur des Aufhellersystems ab, kann sich jedoch, auch je nach Substituenten, zwischen 1 und 4 bewegen. Bevorzugt sind 1 oder 2 derartige Substituenten.

Bevorzugte derartige Substituenten sind z. B. Halogenatome (insbesondere Chlor), $C_1$–$C_4$-Alkyl, Cyclohexyl, $C_3$–$C_4$-Alkenyl, $C_1$–$C_4$-Alkoxy, $C_3$–$C_4$-Alkenyloxy, Phenylsulfonyl, $C_1$–$C_4$-Alkylsulfonyl, $C_2$–$C_5$-Carbalkoxy, Carbamoyl- und Sulfamoylgruppen, wobei die Amidgruppe unsubstituiert sein kann oder mit 1 oder 2 der folgenden Gruppen: $C_1$–$C_4$-Alkyl, $C_1$–$C_4$-Hydroxyalkyl, $C_2$–$C_5$-Cyanoalkyl, $C_1$–$C_4$-Halogenalkyl, Benzyl oder Phenyl substituiert sein kann, wobei die Amidgruppe auch ein gesättigter 5- oder 6-gliedriger Stickstoffheterocyclus, beispielsweise ein Piperidin-, Piperazin-, Morpholin-, Pyrrolidin-, Imidazolidin-, oder Thiomorpholinrest sein kann, der gegebenenfalls durch eine oder 2 Alkyl- oder Hydroxyalkylgruppen substituiert sein kann.

Bevorzugte substituierte Alkylgruppen R, $R_1$ und $R_2$ sind Chloralkyl, Hydroxyalkyl, Cyanoalkyl, Phenylalkyl (insbesondere Benzyl) und Alkoxyalkyl. Für allfällige Substituenten der Amidgruppen in Carbamoyl oder Sulfamoyl siehe vorstehenden Absatz.

Als 5- oder 6-gliedrige gesättigte Heterocyclen, die $R_1$ und $R_2$ gemeinsam mit dem Stickstoffatom bilden können, seien besonders der Piperidin-, Piperazin-, Morpholin-, Thiomorpholin-, Pyrrolidin- und Imidazolidinring zu nennen, die gegebenenfalls noch weiter substituiert sein können mit einer oder zwei Alkylgruppen mit 1 bis 4 C-Atomen.

Als 5- oder 6-gliedrige ungesättigte Heterocyclen, die $R_1$ und $R_2$ gemeinsam mit dem Stickstoffatom bilden können, seien besonders Pyrrolyl-, Imidazolyl-, Pyrazolyl-, 1,2,3-Triazolyl-, 1,2,4-Triazolyl-, Tetrazolyl-, Benzimidazolyl- und Benztriazolylreste erwähnt, die gegebenenfalls mit Halogenatomen wie Chlor oder Brom, Alkylgruppen oder Alkoxygruppen (jeweils bevorzugt mit 1–4 C-Atomen), substituiert sein können. Beispiele hierfür sind Pyrazolyl-(1)-, 4-Methyl-pyrazolyl-(1)-, 3,5-Dimethylpyrazolyl-(1)-, 4-Methoxypyrazolyl-(1)-, 4-Methoxy-äthoxypyrazolyl-(1)-, Imidazolyl-(1)-, 2-Methylimidazolyl-(1)-, Benzimidazolyl-(1)-, 1,2,3-Triazolyl-(1)-, Benztriazolyl-(1)-, 1,2,4-Triazolyl-(1)-, 3,5-Dimethyl-1,2,4-triazolyl-(1)- und 1,2,3,4-Tetrazolyl-(1)-Reste.

Die Alkylen- oder Alkylenoxyalkylengruppe Y kann verzweigt oder geradkettig sein und beispielsweise 1 bis 20 C-Atome aufweisen. Bevorzugt enthält sie 1 bis 12, insbesondere 1 bis 6 C-Atome. Besonders bevorzugt sind unverzweigte Alkylengruppen mit 1 bis 4 C-Atomen.

Für den Fall, dass X = –CON(R)–, oder $-SO_2N(R)-$ und Y = Äthylen oder Propylen, kann R und $R_1$ gemeinsam die Äthylen- oder Methylengruppe bedeuten. Es entstehen so entsprechende, 2 N-Atome enthaltende, gesättigte Heterocyclen, z. B. Gruppierungen der Formeln

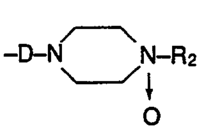 , 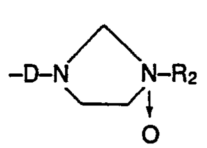 , 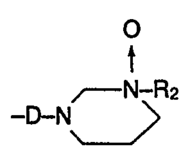 und 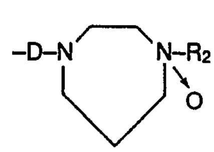 ,

wovon die drei erstgenannten bevorzugt sind, insbesondere die erstgenannte. D bedeutet jeweils CO oder $SO_2$.

Hervorzuheben sind unter den Aminoxidverbindungen solche der Formel (1), worin X ein Sauerstoff- oder Schwefelatom oder eine Gruppe der Formel $-SO_2-$, –CON(R)– oder $-SO_2N(R)-$ und Y eine geradkettige oder verzweigte Alkylengruppe mit 1 bis 12 C-Atomen bedeuten, wobei R wie in Formel (1) definiert ist und R und $R_1$ gegebenenfalls zusammen die unter Formel (1) angegebene Bedeutung haben können.

Praktisch besonders wichtig im Rahmen der Formel (1) sind die Aminoxidverbindungen der Formel

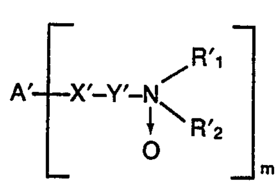 , (2)

worin

A′ einen unsubstituierten oder durch Halogen, Alkyl, Hydroxyalkyl, Halogenalkyl, Cyanoalkyl,

Alkoxyalkyl, Phenylalkyl, Carboxyalkyl, Carbalkoxyalkyl, Alkenyl, Cycloalkyl, Alkoxy, Alkenyloxy, Alkoxycarbonyl, Aminocarbonyl, Alkyl- oder Dialkylaminocarbonyl, Cyano, Alkylsulfonyl, Alkoxysulfonyl, Aminosulfonyl, Hydroxy, Carboxy, Sulfo oder Trifluormethyl substituierten Rest der Formeln

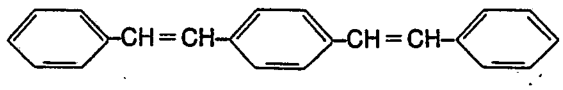

, (3)

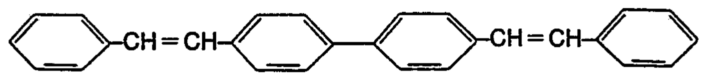

, (4)

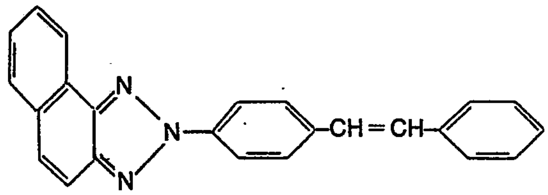

, (5)

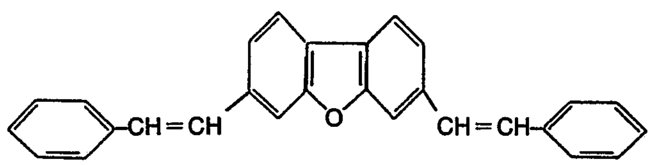

, (6)

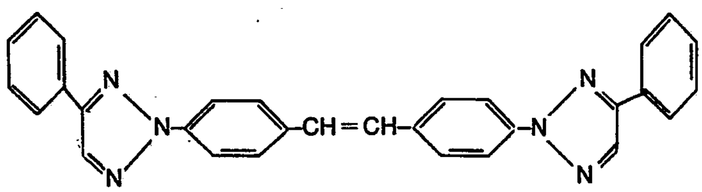

, (7)

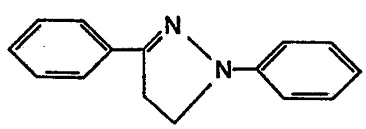

(8) oder

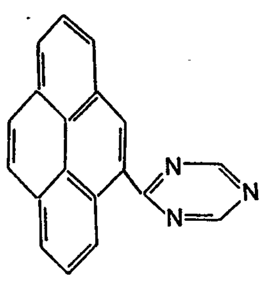

, (8a)

X′ ein Sauerstoffatom oder eine Gruppe der Formel $-SO_2N(R')-$ oder $-SO_2-$, wobei

R′ für Wasserstoff oder unsubstituiertes oder mit Hydroxy, Cyano oder Halogen substituiertes Alkyl mit 1 bis 4 C-Atomen im Alkylteil steht,

Y′ eine geradkettige oder verzweigte Alkylen- oder Alkylenoxyalkylengruppe,

m die Zahl 1 oder 2 und

$R_1'$ und $R_2'$ unabhängig voneinander unsubstituiertes oder mit Halogen, Cyano, Hydroxy, Alkoxy mit 1 bis 4 C-Atomen, Phenyl, Chlorphenyl, Methoxyphenyl, Methylphenyl oder Alkoxycarbonyl mit 2 bis 5 C-Atomen substituiertes Alkyl mit 1 bis 8 C-Atomen im Alkylteil oder $R_1'$ und $R_2'$ zusammen mit dem Stickstoffatom, an das sie gebunden sind, einen 5- oder 6-gliedrigen gesättigten oder ungesättigten Heterocyclus, der gegebenenfalls noch ein oder zwei weitere Heteroatome als Ringglieder enthalten kann, wobei ein gegebenenfalls gesättigter Heterocyclus durch 1 oder 2 $C_1$–$C_4$-Alkylgruppen und ein ungesättigter Heterocyclus durch Halogen, Alkyl oder Alkoxy substituiert sein kann, bedeuten; oder worin für den Fall, dass X′ für eine Gruppe der Formel

$-SO_2N(R')-$ und Y′ für Äthylen oder Propylen stehen, R′ und $R_1'$ gemeinsam die Äthylen- oder Methylengruppe bedeuten, wodurch eine Gruppierung der Formel

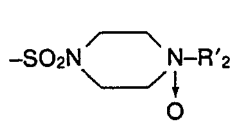
,
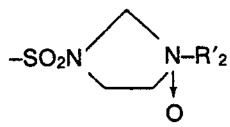
oder
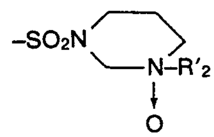
entsteht.

Tragen die Reste A′ Substituenten, so sind dies im Falle der Reste der Formeln (3) und (4) bevorzugt 2 Substituenten, die in der Regel gleich sind und vorzugsweise symmetrisch, insbesondere an den äusseren Phenylringen angeordnet sind. Die übrigen Reste können, falls sie substituiert sind, vorzugsweise 1 bis 2 der genannten Substituenten enthalten.

Unter den Aminoxidverbindungen der Formel (1), die als Aufhellerrest A einen solchen der Formel (3) oder (4) enthalten, seien besonders jene der Formel

(9) 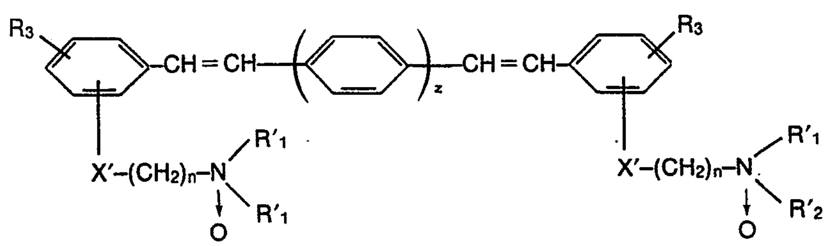
,

worin z für die Zahl 1 oder 2 und n für eine ganze Zahl zwischen 1 und 4 stehen, X′, $R_1'$ und $R_2'$ wie in Formel (2) definiert sind und $R_3$ Halogen, Alkyl mit 1 bis 4 C-Atomen, Cycloalkyl mit 5 oder 6 C-Atomen, Alkoxy mit 1 bis 4 C-Atomen, Alkylsulfonyl mit 1 bis 4 C-Atomen, Phenylsulfonyl, Carbalkoxy mit 2 bis 5 C-Atomen, Carbamoyl oder Sulfamoyl bedeutet, erwähnt.

Von den Verbindungen der Formel (9) sind jene der Formel

(10) 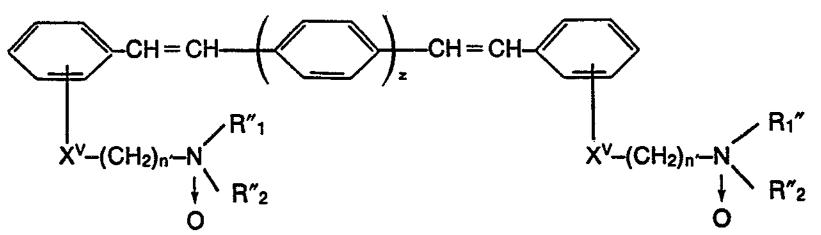
,

worin

z die Zahl 1 oder 2,

$X^v$ ein Sauerstoffatom oder eine Gruppe der Formel $-SO_2N(R')$, wobei R′ für Wasserstoff oder unsubstituiertes oder mit Hydroxy, Cyano oder Halogen substituiertes Alkyl mit 1 bis 4 C-Atomen im Alkylteil steht,

n′ eine ganze Zahl zwischen 1 und 3 und $R_1''$ und $R_2''$ unabhängig voneinander Alkyl oder Hydroxyalkyl mit 1 bis 4 C-Atomen bedeuten, bevorzugt, insbesondere jene der Formel

(11) 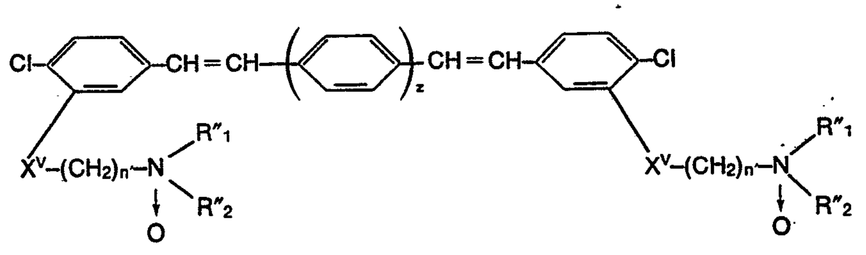
,

worin

z die Zahl 1 oder 2,

$X^v$ ein Sauerstoffatom oder eine Gruppe der Formel $-SO_2N(R')$, wobei R′ für Wasserstoff oder unsubstituiertes oder mit Hydroxy, Cyano oder Halogen substituiertes Alkyl mit 1 bis 4 C-Atomen im Alkylteil steht,

n′ eine ganze Zahl zwischen 1 und 3 und $R_1''$ und $R_2''$ unabhängig voneinander Alkyl oder Hydroxyalkyl mit 1 bis 4 C-Atomen bedeuten, und der Formel

(12) 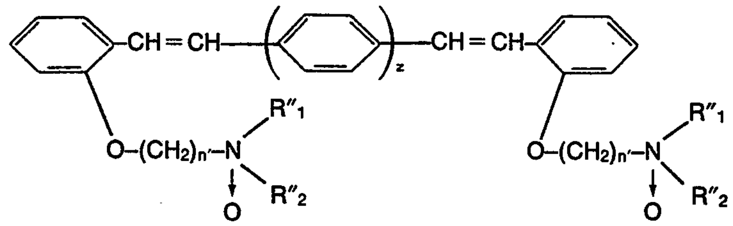 ,

worin z, n', $R_1''$ und $R_2''$ wie in Formel (10) definiert sind.

Unter den Aminoxidverbindungen der Formel (1), die als Aufhellerrest A einen solchen der Formel (8) enthalten, sind besonders jene der Formel

(13) 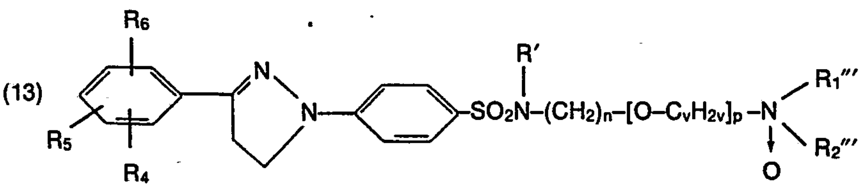 ,

worin

$R_4$ Wasserstoff oder Halogen, $R_5$ und $R_6$ jeweils Wasserstoff, Halogen, Alkyl oder Alkoxy mit je 1 bis 4 C-Atomen, n und v jeweils eine ganze Zahl zwischen 1 und 4, p 0 oder 1, R' Wasserstoff, unsubstituiertes oder mit Hydroxy, Cyano oder Halogen substituiertes Alkyl mit 1 bis 4 C-Atomen im Alkylteil,

$R_1'''$ und $R_2'''$ unabhängig voneinander unsubstituiertes oder mit Halogen, Cyano, Hydroxy, Alkoxy mit 1 bis 4 C-Atomen, Phenyl, Chlorphenyl, Methoxyphenyl, Methylphenyl oder Alkoxycarbonyl mit 2 bis 5 C-Atomen substituiertes Alkyl mit 1 bis 8 C-Atomen im Alkylteil bedeuten oder worin für den Fall, dass n für 2 oder 3 und p für 0 steht, R' und $R_1'''$ gemeinsam die Äthylen- oder Methylengruppe bedeuten, wodurch eine Gruppierung der Formel

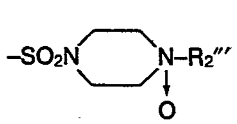 , 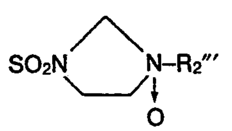 oder 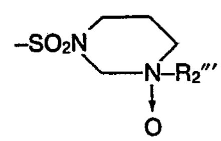

entsteht, zu erwähnen.

Von den Letztgenannten sind die Verbindungen der Formel

(14) 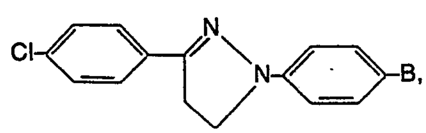

worin B eine Gruppe der Formel

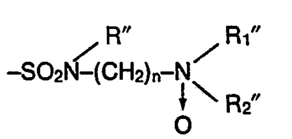 , 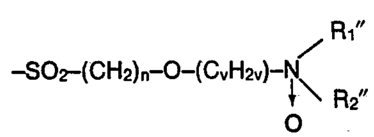

oder

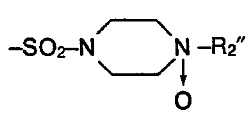

bedeutet, worin R'' für Wasserstoff, Alkyl, Hydroxyalkyl oder Cyanoalkyl mit jeweils 1 bis 4 C-Atomen im Alkylteil, $R_1''$ und $R_2''$ jeweils für Alkyl oder Hydroxyalkyl mit 1 bis 4 C-Atomen und n und v jeweils für eine ganze Zahl zwischen 1 und 4 stehen, bevorzugt.

Für besonders vorteilhafte Verbindungen der Formel (1), in denen A einen Aufhellerrest der Formel (5) darstellt, stehen jene der Formel

(15)

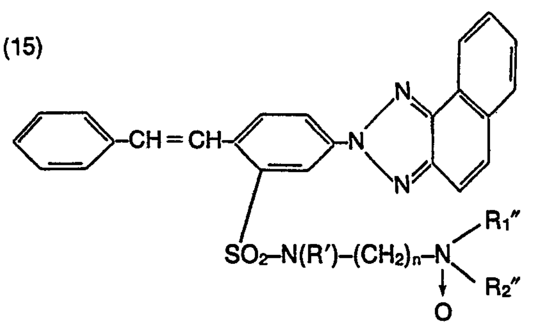

worin R′ Wasserstoff, Alkyl, Hydroxyalkyl, Cyanoalkyl oder Halogenalkyl mit jeweils 1 bis 4 C-Atomen im Alkylteil, $R_1''$ und $R_2''$ jeweils Alkyl oder Hydroxyalkyl mit 1 bis 4 Kohlenstoffatomen und n eine ganze Zahl zwischen 1 und 4 bedeuten.

Von den Verbindungen der Formel (1), in denen A einen Aufhellerrest der Formel (8a) darstellt, seien vor allem solche der Formel

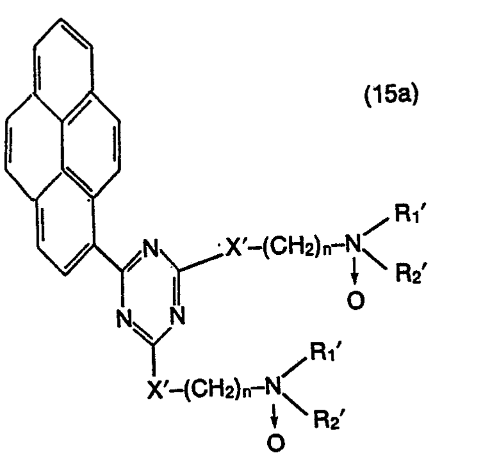

(15a) ,

worin n für eine ganze Zahl zwischen 1 und 4 steht und X′, $R_1'$ und $R_2'$ wie in Formel 2 definiert sind, wobei X′ vorzugsweise Sauerstoff und $R_1'$ und $R_2'$ vorzugsweise unabhängig voneinander Alkyl oder Hydroxyalkyl mit 1 bis 4 C-Atomen bedeuten, erwähnt.

Die erfindungsgemässen Aminoxidverbindungen der Formel (1) können nach an sich bekannten Verfahren hergestellt werden.

So können Verbindungen der Formel (1), in denen X eine direkte Bindung zwischen A und Y, ein Sauerstoffatom oder eine Gruppe der Formel $-SO_2-O-$, $-SO_2-$, $-COO-$, $-CON(R)-$ oder $-SO_2N(R)-$ bedeutet, hergestellt werden, indem man eine Verbindung der Formel

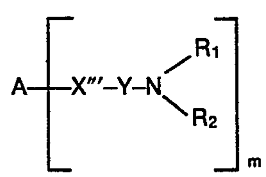

, (16)

worin A, Y, $R_1$, $R_2$ und m wie in Formel (1) definiert sind und X‴ eine direkte Bindung zwischen A und Y, ein Sauerstoff- oder Schwefelatom oder eine Gruppe der Formel $-SO-$, $-SO_2-O-$, $-SO_2-$, $-COO-$, $-CON(R)-$ oder $-SO_2N(R)-$ bedeutet, wobei R ebenfalls die in Formel (1) angegebene Bedeutung hat, oxidiert.

Verbindungen der Formel (16) mit X‴ = $-S-$ oder $-SO-$ werden dabei zu Verbindungen der Formel (1) mit X = $-SO_2-$ oxidiert.

Die Oxidation der Verbindungen der Formel (16) erfolgt vorzugsweise mit einer «Per-Verbindung». Als solche kommen anorganische und organische Verbindungen, die Sauerstoff-Sauerstoff-Bindungen (–O–O–) in ihrem Molekül enthalten, wie z.B. Wasserstoffperoxid, Peroxymonoschwefelsäure (Caro'sche Säure), Ozon, Perameisensäure, Peressigsäure oder gegebenenfalls substituierte (z.B. mit Chlor oder Methyl) Perbenzoesäure in Betracht. Von besonderer praktischer Bedeutung sind Wasserstoffperoxid, Perameisensäure und Peressigsäure. Auch ein Gemisch verschiedener «Per-Verbindungen» kann verwendet werden. Solche Gemische kommen besonders in Frage, wenn als «Per-Verbindung» eine organische Verbindung verwendet wird, die «in situ» durch Oxidation von einer organischen Säure, wie z.B. Ameisensäure oder Essigsäure, mittels einem Überschuss an Wasserstoffperoxid hergestellt wird.

Bei der Verwendung von Wasserstoffperoxid, Perameisensäure oder Peressigsäure werden bevorzugt verdünnte Lösungen dieser «Per-Verbindungen», wie z.B. 30%ige Wasserstoffperoxidlösungen, eingesetzt. Die einzusetzende Oxidationsmittelmenge bewegt sich in weiten Grenzen und richtet sich naturgemäss weitgehend nach der Oxidationskraft der eingesetzten «Per-Verbindung» und nach der Reaktionsfähigkeit des zu oxidierenden Amins. Vorteilhaft verwendet man die äquivalente Menge an «Per-Verbindung»; Überschüsse bis zu einem Vielfachen der äquivalenten Menge können insbesondere zur Erzielung eines raschen Reaktionsablaufs ebenfalls verwendet werden.

Die Oxidation der Ausgangsprodukte der Formel (16) zu den erfindungsgemässen Aminoxidverbindungen der Formel (1) wird zweckmässigerweise in einem unter den Reaktionsbedingungen inerten Lösungsmittel durchgeführt. Als solche Lösungsmittel kommen apolare und dipolare, aprotische und protische Lösungsmittel, wie z.B. Cyclohexan, Toluol, Xylol, Chlorbenzol, Methylenchlorid, 1,2-Dichloräthan, Chloroform, Aceton, Äthylmethylketon, Dioxan, Dimethylformamid, Diäthylformamid, N-Methylpyrrolidon, Äthanol, Isopropanol, Essigsäureanhydrid usw. in Betracht. Auch in Wasser oder in Wasser enthaltenden Gemischen in Gegenwart oder Abwesenheit von Phasentransferkatalysatoren lässt sich die Oxidation durchführen.

Die Oxidation der Ausgangsprodukte der Formel (16) zu den erfindungsgemässen Aminoxidverbindungen der Formel (1) wird vorzugsweise in einem mit Wasser mischbaren organischen Lösungsmittel, in welchem die Ausgangsprodukte der Formel (16) teilweise oder vollständig löslich sind, wie z.B. in Aceton, Äthylmethylketon oder Dioxan, durchgeführt. Die erfindungsgemässen Aminoxidverbindungen der Formel (1) fallen sehr oft während der Oxidation kristallin aus und werden durch Filtration isoliert. Die auf diese Weise in nahezu reinem Zustand isolierten Aminoxidverbindungen der Formel (1) werden vorzugsweise ohne Reinigung als optische Aufheller verwendet.

Die Temperatur während der Oxidation kann sich in Abhängigkeit vom gewählten Oxidationsmittel und vom verwendeten Lösungsmittel in weiten Grenzen bewegen und kann leicht durch Vorversuche ermittelt werden. Die Oxidation der Ausgangsprodukte der Formel (16) zu den erfindungsgemässen Aminoxidverbindungen der Formel (1) wird somit zweckmässig bei Temperaturen zwischen –10 °C und dem Siedepunkt des verwendeten Lösungsmittels durchgeführt; vorzugsweise wird aber die Oxidation bei Temperaturen zwischen 20 und 50 °C durchgeführt.

Die Oxidation der Ausgangsprodukte der Formel (16) zu den erfindungsgemässen Aminoxidverbindungen der Formel (1) kann in gewissen Fällen mit Vorzug in Gegenwart von katalytischen Mengen von anorganischen Per-Verbindungen von säurebildenden Elementen der Gruppen VA, VIA, VIB und VIII des periodischen Systems, wie sie in der US-PS 3 047 579 definiert sind, durchgeführt werden.

Ein weiteres Verfahren zur Herstellung von erfindungsgemässen Verbindungen, nämlich solchen der Formel (1), worin R und $R_1$ gemeinsam keine Ergänzung zu einem Stickstoffheterocyclus bilden können, besteht darin, dass man ein Moläquivalent einer Verbindung der Formel

$$A\text{-}[\text{-}X^{vi}\text{-}H]_m \qquad (17)$$

mit m Moläquivalent einer Verbindung der Formel

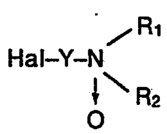 (18)

umsetzt, in welchen Formeln A, m, Y, $R_1$ und $R_2$ wie in Formel (1) definiert sind (mit Ausnahme der gemeinsamen Bedeutung von $R_1+R$), Hal für Chlor oder Brom und $X^{vi}$ für ein Sauerstoff- oder Schwefelatom oder für eine Gruppe der Formel $-SO_2-$ oder –COO– steht.

Zur Herstellung von Verbindungen der Formel (1), worin X eine Gruppe der Formel $-SO_2-O-$ oder –COO– bedeutet, kann man auch ein Moläquivalent einer Verbindung der Formel

$$(19)\ A\text{-}[\text{-}SO_2Hal]_m \quad \text{oder} \quad A\text{-}[\text{-}COHal]_m\ (19a)$$

mit m Moläquivalent einer Verbindung der Formel

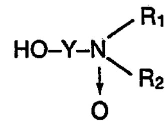 (20)

umsetzen, wobei in den Formeln (19), (19a) und (20) A, m, Y, $R_1$ und $R_2$ wie in Formel (1) definiert sind und Hal für Chlor oder Brom steht.

Die Umsetzung von Verbindungen der Formel (17) mit jenen der Formel (18) bzw. von Verbindungen der Formel (19) oder (19a) mit jenen der Formel (20) wird zweckmässig in einem inerten organischen Lösungsmittel (siehe oben) und in Gegenwart einer schwachen oder starken anorganischen oder organischen Base in an sich bekannter Weise durchgeführt.

Ausgangsverbindungen der Formel (16) sind bekannt, z.B. aus US-PS 4 151 163, BE-PS 722 233, GB-PS 1 186 650, DE-OS 2 921 641 und EP-OSen 19 702 und 19 078 oder können analog den dort beschriebenen Verfahren erhalten werden.

Ausgangsverbindungen der Formel (16), worin X''' eine direkte Bindung zwischen A und Y bedeutet, erhält man vorteilhaft durch Chlormethylierung eines Aufhellersystems A und anschliessender Umsetzung des chlormethylierten Aufhellers mit einem entsprechenden sekundären Amin bzw. mit einem tertiären Amin mit einer funktionellen Gruppe in einer Alkylgruppe. Siehe dazu CH-PS 439 293.

Allgemein kann man die Verbindungen der Formel (16) auch nach dem vorstehend für die Herstellung von Verbindungen der Formel (1) beschriebenen Verfahren erhalten, nämlich durch Umsetzung entweder eines Moläquivalentes einer Verbindung der Formel

$$A\text{-}[\text{-}X'''\text{-}H]_m \qquad (21)$$

mit m Moläquivalent einer Verbindung der Formel

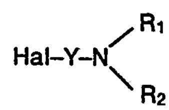 (22)

oder eines Moläquivalentes einer Verbindung der Formel (19) oder (19a) mit m Moläquivalent einer Verbindung der Formel

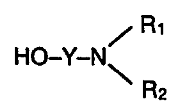 (23),

wobei die allgemeinen Symbole die weiter oben angegebene Bedeutung haben. Die Reaktionsbedingungen sind analog jenen bei der Umsetzung von Verbindungen der Formel (17) mit Verbindungen der Formel (18) bzw. von Verbindungen der Formel (19) oder (19a) mit Verbindungen der Formel (20).

Die Amine der Formeln (22) und (23) sind in der Literatur bekannt und die meisten von ihnen im Handel erhältlich.

Die Aminoxide der Formel (18) und (20) können durch Oxidation von Aminen der Formeln (22) und (23) mittels «Per-Verbindungen» leicht erhalten werden. Solche Oxidationen sind in der Literatur (Houben-Weyl, «Methoden der organischen Chemie», vierte Auflage, Band XI/2, Seite 191–200 (1958); J. Org. Chem. 11, 586–591 (1946); GB-PS 761 001) ausführlich beschrieben.

Die Verbindungen der Formeln (17), (19) und (19a) bzw. (21) sind in der Patentliteratur bekannt oder können nach an sich bekannten Methoden hergestellt werden. So können zum Beispiel solche Verbindungen der Pyrazolin-Klasse nach den CH-PSen 492 069, 386 430 und 475 255 jene der Stilben-Klasse nach der CH-PS 467 819, der US Defensive Publication 778 781, der JA-PS 6 982/69 und der CH-PS 603 513, jene der 1,4-Bis-styrylbenzolklasse nach der DE-OS 2 039 993 und jene der 1,4-Bis-styrylbiphenyl-Klasse nach der CH-PS 513 785 hergestellt werden.

Die oben erwähnten Verfahren zur Herstellung der erfindungsgemässen Verbindungen der Formel (1) gehen von einem schon gebildeten Aufhellergerüst aus, das, wenn es noch keine tertiäre Aminogruppe trägt, entweder zuerst mit m eine tertiäre Amino-Gruppe tragenden Resten verknüpft und dann mit «Per-Verbindungen» zu den Verbindungen der Formel (1) oxidiert wird, oder mit m eine Aminoxid-Gruppe tragenden Resten direkt zu den Verbindungen der Formel (1) verknüpft wird.

In gewissen Fällen kann es aber vorteilhafter sein, die erfindungsgemässen Verbindungen der Formel (1) durch Synthese des Aufhellergerüstes selbst aus Bausteinen, die schon Aminoxid-Gruppen tragen, herzustellen. So können zum Beispiel die erfindungsgemässen Verbindungen der Formel

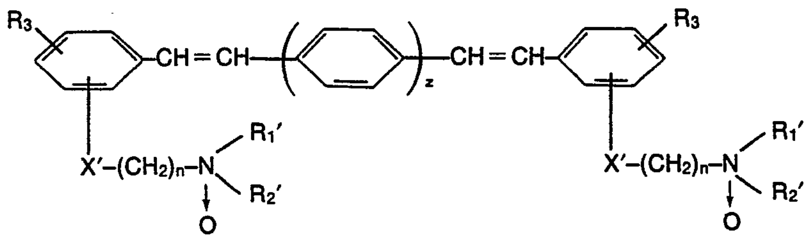

(9)

durch Kondensation von 2 Moläquivalent eines Aminoxids der Formel

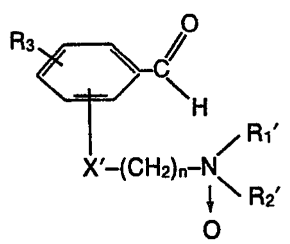

(24)

mit einem Moläquivalent einer Verbindung der Formel

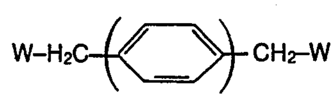

(25)

hergestellt werden, wobei $R_1'$, $R_2'$, $R_3$, X', z und n die in Formel (9) angegebene Bedeutung haben und W einen Rest der Formel –COOV, worin V für Alkyl steht,

–ZnBr, –ZnCl, –MgBr, –MgCl, 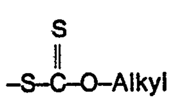 oder 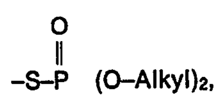

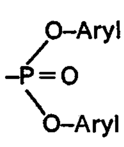 , 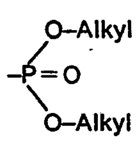 , 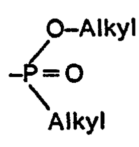 , 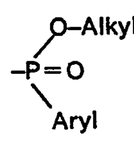 oder 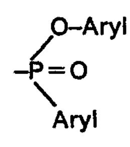

bedeutet.

Die Aminoxide der Formel (24) lassen sich durch Oxidation der in der Literatur bekannten Amine der Formel

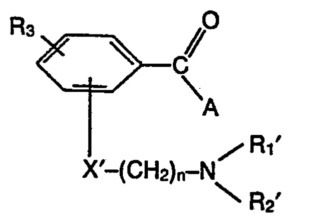

(26)

mit «Per-Verbindungen» leicht herstellen. Die Verbindungen der Formel (25) sind aus der Literatur bekannt.

Die vorstehend definierten neuen Verbindungen der Formel (1) zeigen in gelöstem oder feinverteiltem Zustande eine mehr oder weniger ausgeprägte Fluoreszenz. Sie werden daher zum optischen Aufhellen der verschiedensten synthetischen, halbsynthetischen oder natürlichen organischen Materialien verwendet.

Hierfür seien beispielsweise, ohne dass durch die nachfolgende Übersicht irgendeine Beschränkung hierauf ausgedrückt werden soll, die folgenden Gruppen von organischen Materialien, soweit eine optische Aufhellung derselben in Betracht kommt, genannt:

I. Synthetische organische hochmolekulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungs- oder Abbauprodukte, Polymerisat-Verschnitte oder durch Modifizierung reaktionsfähiger Gruppen erhaltene Produkte, beispielsweise Polymerisate auf Basis von $\alpha,\beta$-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga), von Olefin-Kohlenwasserstoffen (wie z.B. Äthylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),

b) Polymerisationsprodukte, die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis bi- oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Äthylenglykolterephthalsäure-Polyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Polyhexamethylendiamin-adipat), Maleinatharze, Melaminharze, deren Vorkondensate und Analoga, Polycarbonat, Silikone,

d) Polyadditionsprodukte wie Polyurethane (vernetzt und unvernetzt), Epoxidharze.

II. Halbsynthetische organische Materialien, z.B. Celluloseester verschiedener Veresterungsgrade (sogenanntes $2^1/_2$-Acetat, Triacetat) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d.h. beispielsweise als vorwiegend dreidimensional ausgedehnte Körper wie Platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lakke, Überzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als Pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflokkungs-Gebilde oder als textile Gewebe oder textile Verbundstoffe, Gewirke sowie als Papiere, Pappen oder Papiermassen vorliegen.

Den erfindungsgemäss anzuwendenden Verbindungen kommt u.a. Bedeutung für die Behandlung von textilen organischen Materialien, insbesondere textilen Geweben, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wässerigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140 °C, beispielsweise bei Siedetemperatur des Bades oder in deren Nähe (etwa 90 °C), durchgeführt. Für die erfindungsgemässe Veredlung textiler Substrate kommen auch Lösungen oder Emulsionen in organischen Lösungsmitteln in Betracht, wie dies in der Färbereipraxis in der sogenannten Lösungsmittelfärberei (Foulard-Thermofixierapplikation, Ausziehfärbeverfahren in Färbemaschinen) praktiziert wird.

Die neuen erfindungsgemässen optischen Aufheller können beispielsweise auch zur Aufhellung von Papiermassen, unter anderem auch in Gegenwart von z.B. kationischen Retentionsmitteln und anderen Zusatzstoffen eingesetzt werden.

Die neuen optischen Aufhellmittel gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der Pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

- Zugabe zu den Ausgangssubstanzen (z.B. Monomeren, oder Zwischenprodukten (z.B. Vorkondensaten, Praepolymeren), d.h. vor oder während der Polymerisation, Polykondensation oder Polyaddition,
- Aufpudern auf Polymerisatschnitzel oder Granulate für Spinnmassen,
- Badfärbung von Polymerisatschnitzeln oder Granulaten für Spinnmassen,
- Dosierte Zugabe zu Spinnschmelzen oder Spinnlösungen,
- Applikation auf Spinnkabel vor dem Verstrekken.

Die neuen optischen Aufhellmittel gemäss vorliegender Erfindung können beispielsweise auch in folgenden Anwendungsformen eingesetzt werden:

a) Mischungen mit Farbstoffen (Nuancierung) oder Pigmenten (Farb- oder insbesondere z.B. Weisspigmenten) oder als Zusatz zu Färbebädern, Druck-, Ätz- oder Reservepasten. Ferner auch zur Nachbehandlung von Färbungen, Drukken oder Ätzdrucken,

b) in Mischungen mit sogenannten «Carriern», Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie «wash- and -wear», «permanent-press», «noiron»), ferner Flammfest-, Weichgriff-, Schmutzablöse («anti-soiling»)- oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu den verschiedensten industriellen Produkten, um dieselben marktfähiger zu machen (z.B. Aspektverbesserung von Seifen, Waschmitteln, Pigmenten),

f) in Kombination mit anderen, optisch aufhellend wirkenden Substanzen,

g) in Spinnbadpräparationen, d.h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser, z.B. als Nachbehandlung von nassversponnenen Polyacrylfasern im sogenannten Gelzustand,

h) als Scintillatoren, für verschiedene Zwecke photographischer Art, wie z.B. für elektrophotographische Reproduktion oder Supersensibilisierung,

i) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische oder eine kombinierte chemisch/thermische Behandlung darstellen. So verfährt man beispielsweise bei der optischen Aufhellung einer Reihe von Fasersubstraten, z.B. von Polyesterfasern, mit den erfindungsgemässen Aufhellern zweckmässig in der Weise, dass man diese Fasern mit den wässrigen Dispersionen (gegebenenfalls auch Lösungen) der Aufhellmittel bei Temperaturen unter 75 °C, z.B. bei Raumtemperatur, imprägniert und einer trockenen Wärmebehandlung bei Temperaturen über 100 °C unterwirft, wobei es sich im allgemeinen empfiehlt, das Fasermaterial vorher noch bei mässig erhöhter Temperatur, z.B. bei mindestens 60 °C bis etwa 130 °C zu trocknen. Die Wärmebehandlung in trockenem Zustande erfolgt dann vorteilhaft bei Temperaturen zwischen 120 und 225 °C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z.B. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder Kaliumtripolyphosphaten oder Alkalimetallsilicaten.

Die neuen optischen Aufhellmittel eignen sich auch besonders als Zusätze für Waschbäder oder zu Gewerbe- und Haushaltswasch- und Wäschenachbehandlungsmitteln, wobei sie in verschiedener Weise zugesetzt werden können. Zu Waschbädern werden sie zweckmässig in Form ihrer Lösungen in Wasser oder organischen Lösungsmitteln oder auch in feiner Verteilung als wässerige Dispersionen zugegeben. Zu Haushalt- oder gewerblichen Waschmitteln werden sie vorteilhaft in irgend einer Phase des Herstellungsprozesses der Waschmittel, z.B. der sogenannten «slurry» vor dem Zerstäuben dem Waschpulver oder bei der Vorbereitung flüssiger Waschmittelkombinationen, zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise mit den waschaktiven Substanzen vermischen, verkneten oder vermahlen und so dem fertigen Waschpulver zumischen. Sie können jedoch auch gelöst oder vordispergiert auf das fertige Waschmittel aufgesprüht werden.

Als Waschmittel kommen beispielsweise die bekannten Mischungen von Waschaktivsubstanzen wie beispielsweise Seife in Form von Schnitzeln und Pulver, Synthetika, lösliche Salze von Sulfonsäurehalbestern höherer Fettalkohole, höher und/oder mehrfach alkyl-substituierten Arylsulfonsäuren, Sulfocarbonsäureester mittlerer bis höherer Alkohole, Fettsäureacylaminoalkyl- oder -aminoarylglycerinsulfonate, Phosphorsäureester von Fettalkoholen usw. in Frage. Als Aufbaustoffe, sogenannte «Builders», kommen z.B. Alkalipoly- und -poly-metaphosphate, Alkalipyrophosphate, Alkalisalze der Carboxymethylcellulose und andere «Soilredepositionsinhibitoren», ferner Alkalisilikate, Alkalicarbonate, Alkaliborate, Alkaliperborate, Nitrilotriessigsäure, Äthylendiaminotetraessigsäure, Schaumstabilisatoren wie Alkanolamide höherer Fettsäuren, in Betracht. Ferner können in den Waschmitteln beispielsweise enthalten sein:

antistatische Mittel, rückfettende Hautschutzmittel wie Lanolin, Enzyme, Antimikrobika, Parfüme und Farbstoffe.

Die erfindungsgemässen Verbindungen werden in Mengen von 0,005–1% oder mehr, bezogen auf das Gewicht des flüssigen oder pulverförmigen, fertigen Waschmittels, zugesetzt. Waschflotten, die die angegebenen Mengen der beanspruchten optischen Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt:

Die angegebenen Textilien werden während 1 bis 30 Minuten bei 20 bis 100 °C in einem Waschbad behandelt, das 1 bis 10 g/kg eines aufgebauten, zusammengesetzten Waschmittels und 0,05 bis 1%, bezogen auf das Waschmittelgewicht, der beanspruchten Aufhellmittel enthält. Das Flottenverhältnis kann 1:3 bis 1:50 betragen. Nach dem Waschen wird wie üblich gespült und getrocknet. Das Waschbad kann als Bleichzusatz 0,2 g/l Aktivchlor (z.B. als Hypochlorit) oder 0,1 bis 2 g/l Natriumperborat enthalten.

Die erfindungsgemässen Verbindungen können auch im Nachspülbad, wie es zur blossen Verleihung von Weichgriff, antistatischen Eigenschaften, Antisoileffekten, Duftnoten usw. üblich ist, eingesetzt werden. Insbesondere eignen sie sich für den Einsatz in Wäschenachbehandlungsmitteln, welche kationische Weichmacher enthalten.

Die neuen optischen Aufheller eignen sich auch sehr gut als Aufheller für konzentrierte flüssige Waschmittel, welche nichtionogene Tenside und kationische Weichmacher oder Tenside enthalten.

Die vorliegende Erfindung betrifft daher auch ein Waschmittel, welches vorzugsweise in flüssiger Form vorliegt und welches neben den neuen Aminoxidverbindungen und den üblichen Zusätzen noch nichtionogene Tenside und kationische Textilweichmacher enthält.

Als nicht-ionogene Tenside kommen die im Handel üblichen in Betracht, z.B. die wasserlöslichen Produkte, die aus der Addition eines Alkylenoxides bzw. einer äquivalenten Verbindung mit einem reaktiven Wasserstoffatom einer hydrophoben Verbindung erhalten werden. Die hydrophoben organischen Produkte können Heterocyclen und besonders Aliphaten oder Aromaten sein. Bevorzugt sind höhere aliphatische Alkohole und Alkylphenole, wobei aber auch andere, wie z.B. Carbonsäuren, Carboxamide, Mercaptane, Sulfamide usw. verwendet werden können. Bevorzugte nichtionogene Verbindungen sind die Additionsprodukte von Äthylenoxid mit höheren aliphatischen Alkoholen mit 6 bis 50 und mehr C-Atomen. Die Menge Äthylenoxid kann sich in weiteren Grenzen bewegen, aber im allgemeinen werden zumindest 5 Mol Äthylenoxid pro Mol hydrophober Substanz gebraucht. Anstelle von Äthylenoxid können ganz oder teilweise andere niedere Alkylenoxide, wie z.B. Propylenoxid und Butylenoxid verwendet werden. Als weitere nichtionogene Verbindungen kommen in Betracht:

a) Polyoxyalkylenester organischer Säuren, wie höherer Fettsäuren, Harzsäuren, Talgölsäuren und Säuren der Oxidationsprodukte des Erdöls, deren Ester in der Regel im Säureteil 10 bis 22 C-Atome aufweisen und ca. 12 bis ca. 30 Mole Äthylenoxid oder dessen Äquivalent enthalten.

b) Alkylenoxidaddukte höherer Fettsäureamide, wobei der Fettsäureteil in der Regel 8 bis 22 C-Atome aufweist und mit 10 bis 50 Mol Äthylenoxid kondensiert ist. Die entsprechenden Carboxamide und Sulfamide können ebenfalls als weitgehend äquivalent verwendet werden.

Bei der Herstellung von flüssigen konzentrierten Waschmitteln werden als nichtionogene Tenside vorzugsweise oxalkylierte höhere aliphatische Alkohole verwendet, wobei die Fettalkohole mindestens 6 und vorzugsweise mindestens 8 C-Atome aufweisen. Bevorzugte Alkohole sind Lauryl-, Myristyl-, Cetyl-, Stearyl- und Oleylalkohol, welche mit mindestens 6 Mol Äthylenoxid kondensiert werden. Als typisches nichtionogenes Produkt sei das Additionsprodukt von einem aliphatischen Alkohol mit 12–13 C-Atomen mit ca. 6,5 Mol Äthylenoxid erwähnt. Die entsprechenden Alkylmercaptane sind, nach Kondensation mit Äthylenoxid, ebenfalls als nichtionogene Tenside verwendbar.

Die alkoxylierten höheren aliphatischen Alkohole sind besonders für Haushaltswaschmittel geeignet, da sie leicht biologisch abbaubar sind und gut mit kationischen Tensiden und Textilweichmachern und den übrigen Zusätzen verträglich sind.

Als kationische Textilweichmacher kommen neben anderen vor allem quaternäre Derivate des Ammoniaks und/oder des Imidazolins mit 2 langkettigen, aliphatischen gesättigten oder ungesättigten Resten in Betracht.

Von diesen seien beispielsweise angeführt:

1. Quaternäre Ammoniumsalze der Formel

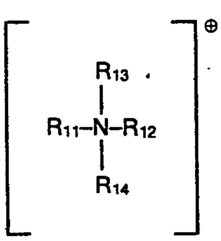

$B^{\ominus}$ , (30)

worin $R_{11}$ Wasserstoff oder eine aliphatische Gruppe mit 1 bis 22 C-Atomen, $R_{12}$ eine aliphatische Gruppe mit 10 bis 22 C-Atomen, $R_{13}$ und $R_{14}$ unabhängig voneinander Alkyl mit 1 bis 4 C-Atomen und $B^{\ominus}$ ein Anion bedeuten. Das Anion $B^{\ominus}$ ist ein beliebiges Anion, das in der Regel durch die Quaternierung eingeführt wird. Vorzugsweise bedeutet es ein Halogenion (inklusive $J^{\ominus}$), ein Alkylsulfat-, Alkansulfonat- oder Arylsulfonation, z.B. das Phenylsulfat-, p-Tolylsulfat- und das p-Chlorphenylsulfonation. Es kann aber auch ein Sulfat-, Sulfit-, Carbonat-, Phosphat-, Nitrat-, Acetat-, Oxalat-, Citrat-, Lactation oder ein anderes Anion einer organischen Carbonsäure sein.

Beispiele für quaternäre Ammonium-Weichmacher sind:
Tallyltrimethylammoniumchlorid,
Ditallyldimethylammoniumchlorid;
Ditallyldimethylammoniumsulfat,
Dihexadecyldimethylammoniumchlorid,
Dioctadecyldimethylammoniumchlorid,
Dieicosyldimethylammoniumchlorid,
Didocosyldimethylammoniumchlorid,
Dihexadecyldiäthylammoniumchlorid,
Dihexadecylmethylammoniumacetat,
Ditallyldipropylammoniumphosphat,
Ditallyldimethylammoniumnitrat,
Dicocoyldimethylammoniumchlorid.

2. Quaternäre Imidazoliniumsalze der Formel

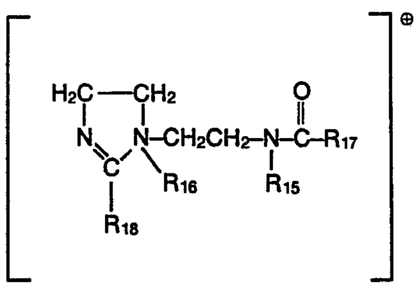

$B^{\ominus}$

(31) ,

worin $R_{15}$ Wasserstoff oder Alkyl mit 1 bis 4 C-Atomen, $R_{16}$ Alkyl mit 1 bis 4 C-Atomen, $R_{17}$ Alkyl mit 1 bis 22 C-Atomen, $R_{18}$ Wasserstoff oder Alkyl mit 1 bis 22, vorzugsweise 15–22 C-Atomen und $B^{\ominus}$ ein Anion bedeuten.

Das Anion $B^{\ominus}$ ist wie in Formel (30) definiert.

Bevorzugt sind solche Verbindungen der Formel (31), worin $R_{17}$ und $R_{18}$ jeweils Alkyl mit 12 bis 22 C-Atomen bedeuten.

Beispiele für bevorzugte Imidazoliniumverbindungen der Formel (31) sind:
1-Methyl-1-stearoylamidoäthyl-2-heptadecyl-imidazoliniummethosulfat,
1-Methyl-1-palmitoylamidoäthyl-2-octadecyl-imidazoliniumchlorid,
2-Tallyl-1-methyl-1-talloylamidoäthyl-imidazoliniummethosulfat.

Weiters sind als Textilweichmacher beispielsweise geeignet:
1-Methyl-1-oleylamidoäthyl-2-oleyl-imidazolinium.
$X^{\ominus}$, 1-Methyl-1-talloylamidoäthyl-2-tallyl-imidazolinium.
$X^{\ominus}$, Di-tallyl-dimethylammonium. $X^{\ominus}$ oder eine Verbindung der Formel

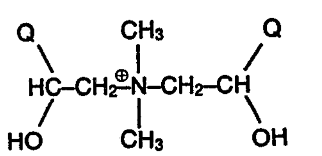

$.X^{\ominus}$ , (32)

worin Q $C_{14}$–$C_{16}$-Alkyl bedeutet und $X^{\ominus}$ für ein Chlorid-, Bromid-, Methylsulfat-, Äthylsulfat-, Methan-, Äthan- oder Toluolsulfonatanion steht.

Von den kationischen Textilweichmachern eignen sich besonders quaternäre Imidazoliniumverbindungen wie das
1-Methyl-1-oleylamidoäthyl-2-oleyl-imidazoliniummethosulfat
und das
1-Methyl-1-talloylamidoäthyl-2-tallyl-imidazoliniummethosulfat.

Die genannten quaternären Textilweichmacher und besonders die zwei letzterwähnten verleihen dem Gewebe einen weichen und flaumigen Griff und gleichzeitig eine gute Wiederanfeuchtbarkeit. Diese Textilweichmacher sind substantiv zum Gewebe und tragen zur Verminderung der statischen Aufladung und der Neigung zum Knittern bei, so dass das Gewebe leichter gebügelt werden kann und angenehmer zu tragen ist.

Das flüssige Medium für die erfindungsgemässen Waschmittel ist wässerig und kann aus Wasser allein oder aus Wasser und zusätzlichen Lösungsmitteln für gewisse Zusätze bestehen. Die zusätzlichen Lösungsmittel können bis zu 20, vorzugsweise bis 15% des gesamten Lösungsmittelanteils ausmachen. Als solche kommen in Betracht: niedere Alkanole oder ein niederes Diol oder Polyol wie z.B. Äthanol, Isopropanol, Äthylenglykol, Propylenglykol und Glycerin. Auch verätherte Polyole wie Diäthylenglykol, Äthylenglykoldimethyläther und Äthylenglykolmonoäthyläther können als zusätzliche Lösungsmittel verwendet werden.

Das erfindungsgemässe flüssige Waschmittel kann verschiedene ausgewählte verträgliche Zusätze enthalten, wie Schmutzsuspendiermittel oder Vergrauungsinhibitoren, z.B. Polyvinylalkohol, Hydroxypropylmethylcellulose; Schauminhibitoren, Konservierungsmittel, z.B. Natriumbenzoat; UV-Absorber und Parfüme. Diese werden selbstverständlich so gewählt, dass sie mit den Hauptkomponenten des Waschmittels verträglich sind.

Die nichtionogenen Tenside werden in Mengen von 25 bis 70 Gewichts-%, vorzugsweise etwa 60 Gewichts-% eingesetzt. Die Konzentration des Textilweichmachers beträgt 5 bis 30 Gewichts-%, vorzugsweise etwa 21 Gewichts-%. Das wässrige Lösungsmittel, vorzugsweise Wasser, das noch mono- di- und mehrwertige Alkohole und ähnliche Lösungsmittel enthalten kann, ist in einer Menge von 5 bis 60 Gewichts-% enthalten. Das fertige flüssige Waschmittel enthält die erfindungsgemässen Verbindungen in Mengen von 0,005 bis 3 Gewichts-%. Der Gehalt an übrigen Hilfsmitteln beträgt vorzugsweise weniger als 5 Gewichts-% des Waschmittels, da die Verwendung von grösseren Mengen die Eigenschaften flüssiger Waschmittel beeinflussen kann. Obschon die bevorzugte erfindungsgemässe waschaktive Zubereitung eine stabile, klare Flüssigkeit ist, kann man ihr ein verträgliches Trübungsmittel hinzugeben, um einen opaken Aspekt hervorzurufen.

Das erfindungsgemässe Waschmittel kann in weichem oder angemessen hartem Wasser bei höherer Temperatur zur Anwendung gelangen. Dieses Waschmittel kann auch zum Waschen von Textilien in sehr hartem Wasser bei tieferer Temperatur verwendet werden. Die Wasserhärte kann deshalb von 0 bis über 300 ppm, berechnet als Calciumcarbonat, schwanken und die Waschtemperatur kann 4 bis 60 °C betragen.

Das erfindungsgemässe Waschmittel löst sich sehr leicht in kaltem oder warmem Waschwasser, reinigt gründlich, eliminiert die statische Aufladung und macht die Wäsche weich, ohne sie zu hydrophobieren. Das bevorzugte Waschmittel liegt als eine klare, stabile Flüssigkeit vor, die ihre Aktivität und Uniformität über eine längere Zeitspanne behält. Zur Herstellung von klaren flüssigen Waschmitteln kann die Konzentration der Aktivsubstanzen nur innerhalb gewisser Grenzen variiert werden. So soll z.B. die Konzentration des Textilweichmachers nicht viel höher sein als 30%, wenn man ein klares flüssiges Waschmittel erhalten will.

Die erfindungsgemässen Verbindungen werden in Mengen von 0,005 bis 1% oder mehr, bezogen auf das Gewicht des flüssigen oder pulverförmigen, fertigen Waschmittels bzw. Textilbehandlungsmittels, zugesetzt. Wasch-/Behandlungsflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc., diesen einen brillanten Aspekt am Tageslicht.

Die Waschbehandlung wird beispielsweise wie folgt durchgeführt:

Die angegebenen Textilien werden während 1 bis 30 Minuten bei 20 bis 100 °C in einem Waschbad behandelt, das 0,1 bis 10 g/kg des jeweiligen Waschmittels und 0,01 bis 1%, bezogen auf das Waschmittelgewicht, der beanspruchten Aufhellmittel enthält. Das Flottenverhältnis kann 1:3 bis 1:50 betragen. Nach dem Waschen wird wie üblich gespült und getrocknet.

In den Beispielen sind Teile, soweit nicht anders angegeben immer Gewichtsteile und Prozente immer Gewichtsprozente. Schmelz- und Siedepunkte sind, sofern nicht anders vermerkt, unkorrigiert.

Beispiel 1:

20,6 g der Verbindung der Formel

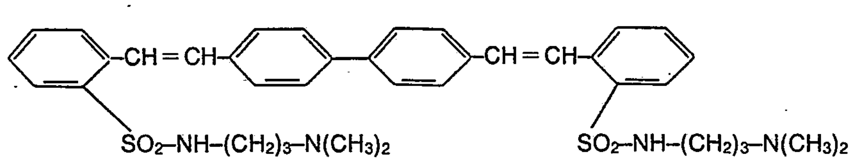

(101)

werden in 500 ml Äthylmethylketon heiss gelöst und bei 40 °C mit 70 g 30%igem Wasserstoffperoxid langsam versetzt. Das Reaktionsgemisch wird nun 60 Stunden bei Raumtemperatur weitergerührt, wobei das Reaktionsprodukt kristallin ausfällt. Das Reaktionsprodukt wird abgenutscht, mit 50 ml Äthylmethylketon gewaschen und unter Vakuum bei 70 °C bis zum konstanten Gewicht getrocknet. Man erhält 20 g (etwa 93% der Theorie) der Verbindung der Formel

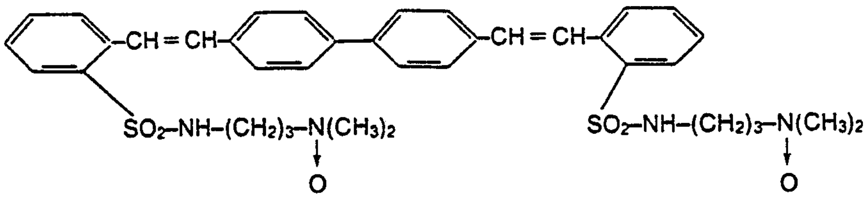

(102)

in Form eines blassgelben kristallinen Pulvers, das bei 192 bis 194 °C unter Zersetzung schmilzt. Nach einmaligem Umkristallisieren aus Alkohol/Wasser erhält man 18 g der Verbindung (102) in Form blassgelber Plättchen mit einem Schmelzpunkt von 193–195 °C (Zersetzung).

Ausgehend von den entsprechenden Aminen werden in analoger Weise die Aminoxid-Verbindungen der Formel

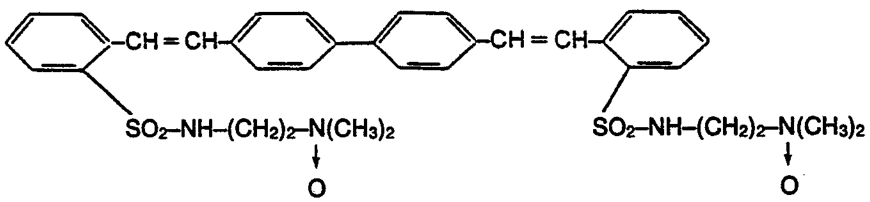

(103)

in Form von gelben Nädelchen (aus Äthanol/Wasser), die bei 182 bis 186 °C unter Zersetzung schmelzen, und der Formel

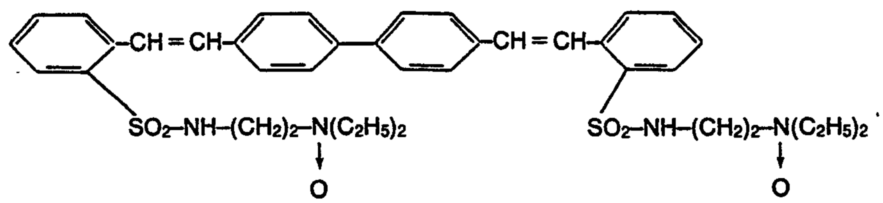

(104).

in Form von blassgelben Plättchen, die bis 164 bis 167 °C unter Zersetzung schmelzen, erhalten.

Die Herstellung der Ausgangsverbindung der Formel (101) ist in Beispiel 8 der britischen Patentschrift Nr. 1 247 934 beschrieben. Homologe Verbindungen werden in analoger Weise hergestellt.

Beispiel 2:

28 g der Verbindung der Formel

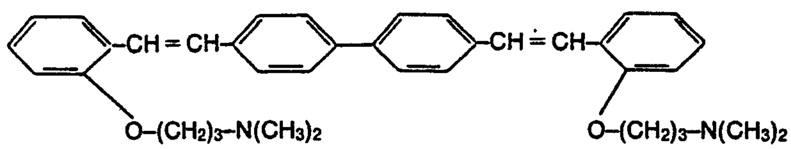

(201)

werden in 500 ml Äthylmethylketon heiss gelöst und bei 50 °C innerhalb 10 Minuten mit 113 g 30%igem Wasserstoffperoxid versetzt. Das Reaktionsgemisch wird nun 48 Stunden bei Raumtemperatur weitergerührt, wobei das Reaktionsprodukt kristallin ausfällt.

Das Reaktionsprodukt wird abgenutscht, mit 50 ml Äthylmethylketon gewaschen und unter Vakuum bei 70 °C bis zum konstanten Gewicht getrocknet.

Man erhält 28 g (etwa 95% der Theorie) der Verbindung der Formel

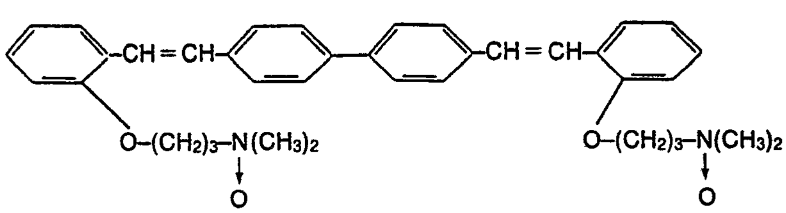

(202)

in Form eines gelben kristallinen Pulvers, das bei 170 bis 172 °C schmilzt.

Nach einmaligem Umkristallisieren aus Alkohol erhält man 20,5 g der Verbindung der Formel (202) als blassgelbe Nädelchen mit einem Schmelzpunkt von 172–173 °C.

Ausgehend von den entsprechenden Aminen werden in analoger Weise die Aminoxid-Verbindungen der Formel

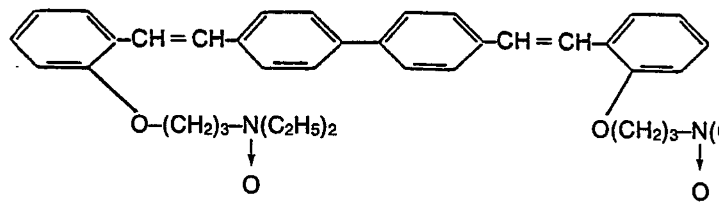

(203)

in Form von gelben Nädelchen, die bei 143 bis 145 °C unter Zersetzung schmelzen, und der Formel

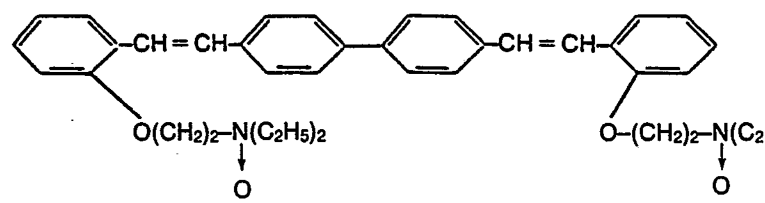

(204)

in Form von blassgelben Plättchen (aus Alkohol), die bei 160 bis 162 °C schmelzen, erhalten.

Die Herstellung der Ausgangsverbindung der Formel (201) ist in der offengelegten EP-Patentanmeldung Nr. 19 702 beschrieben, homologe Verbindungen werden in analoger Weise hergestellt.

Beispiel 3:

18,8 g der Verbindung der Formel

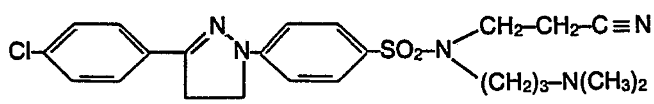

(301)

werden in 300 ml Äthylmethylketon gelöst und bei Raumtemperatur innerhalb 10 Minuten mit 45 g 30%igem Wasserstoffperoxid versetzt. Das Reaktionsgemisch wird nun 48 Stunden bei Raumtemperatur weitergerührt, wobei das Reaktionsprodukt in Lösung bleibt. Die erhaltene klare Reaktionslösung wird nun unter Kühlung mit 2 g Palladium-Kohle (5% Pd) versetzt und über Nacht bei Raumtemperatur weitergerührt, wobei sich der Wasserstoffperoxidüberschuss unter Sauerstoffentwicklung zersetzt.

Das Reaktionsgemisch wird durch Filtration von der Palladium-Kohle befreit und die erhaltene klare Reaktionslösung wird nun mit 500 ml Wasser versetzt und am Rotationsverdampfer unter Vakuum von Äthylmethylketon befreit.

Eine Probe der wässrigen Reaktionslösung wird mit Natriumthiosulfat auf Wasserstoffperoxid titriert, wobei kein Wasserstoffperoxid mehr festgestellt wird. Die wässrige Reaktionslösung wird nun am Rotationsverdampfer unter Vakuum zur Trockne eingeengt und das Reaktionsprodukt aus Alkohol unter Entfärbung mit 5 g Aktivkohle umkristallisiert. Man erhält 15 g (etwa 68% der Theorie) der Verbindung der Formel

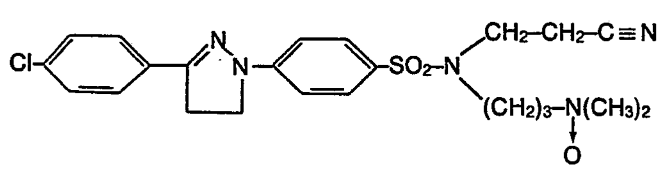

$\cdot CH_3-CH_2-OH$
$\cdot H_2O$

(302)

in Form von gelben Nädelchen, die bei 128 bis 133 °C unter Zersetzung schmelzen. Nach Umkristallisieren aus Essigester/Methanol erhält man 10,5 g der Verbindung der Formel (302) in Form gelber Nädelchen mit einem Schmelzpunkt von 136–138 °C (Zersetzung).

Die Herstellung der Ausgangsverbindung der Formel (301) ist in der britischen Patentschrift Nr. 2 000 507 beschrieben.

Beispiel 4:

21 g der Verbindung der Formel

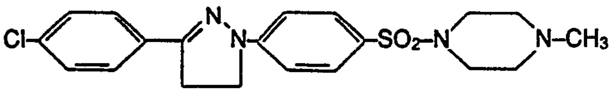

(401)

werden in 300 ml Chloroform gelöst und bei Raumtemperatur innerhalb 15 Minuten mit einer Lösung von 10 g 85%iger m-Chlorperbenzoesäure in 100 ml Chloroform versetzt. Das Reaktionsgemisch wird nun zuerst 24 Stunden bei Raumtemperatur und dann 3 Stunden am Rückfluss weitergerührt. Die erhaltene klare Reaktionslösung wird am Rotationsverdampfer unter Vakuum zur Trockene eingeengt und das Reaktionsprodukt aus Alkohol umkristallisiert. Man erhält 26 g (etwa 88% der Theorie) der Verbindung der Formel

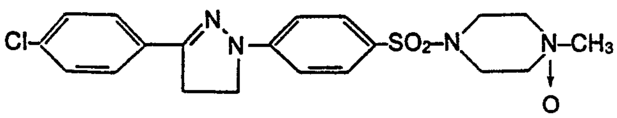

·

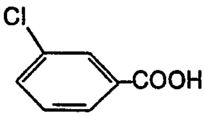

(402)

in Form von gelben Nädelchen, die bei 161 bis 167 °C unter Zersetzung schmelzen. Nach zweimaligem Umkristallisieren aus Alkohol erhält man 21 g der Verbindung der Formel (402) in Form gelber Nädelchen mit einem Schmelzpunkt von 165–166 °C (Zersetzung).

Die Herstellung der Ausgangsverbindung der Formel (401) ist in Beispiel 4 der britischen Patentschrift Nr. 1 186 650 beschrieben.

Beispiel 5:

18 g der Verbindung der Formel

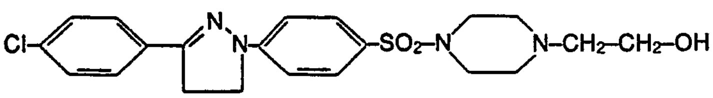

(501)

werden in 400 ml Chloroform gelöst und bei Raumtemperatur innerhalb 15 Minuten mit einer Lösung von 8 g 85%iger m-Chlorperbenzoesäure in 80 ml Chloroform versetzt. Das Reaktionsgemisch wird nun zuerst 24 Stunden bei Raumtemperatur und dann 3 Stunden unter Rückfluss weitergerührt. Die erhaltene klare Reaktionslösung wird am Rotationsverdampfer unter Vakuum zur Trockne eingeengt und das Reaktionsprodukt aus Alkohol umkristallisiert. Man erhält 26 g (etwa 84% der Theorie) der Verbindung der Formel

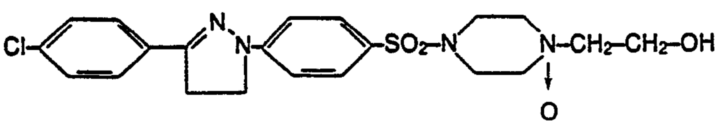

·

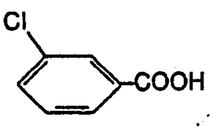

(502)

in Form von gelben Nadeln, die bei 148 bis 150 °C unter Zersetzung schmelzen. Nach einmaligem Umkristallisieren aus Alkohol erhält man 22 g der Verbindung der Formel (502) als gelbe Nadeln mit einem Schmelzpunkt von 152–154 °C (Zersetzung).

Löst man nun diese Verbindung in heissem Alkohol und behandelt die Lösung mit Aktivkohle, dann erhält man 14 g der freien Base der Formel

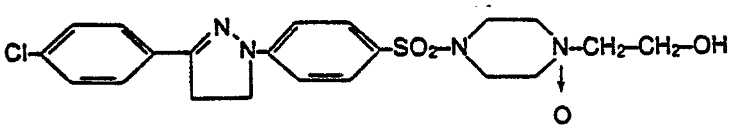

(503)

in Form von gelben Nädelchen, die bei 196 bis 199 °C unter Zersetzung schmelzen.

Die Herstellung der Ausgangsverbindung der Formel (501) ist in Beispiel 1 der britischen Patentschrift Nr. 1 186 650 beschrieben.

Beispiel 6:

20 g der Verbindung der Formel

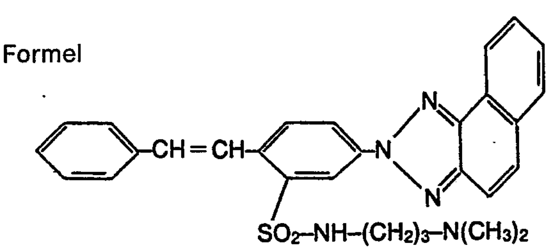

(601)

werden in 400 ml Äthylmethylketon gelöst und bei Raumtemperatur mit 45 g 30%igem Wasserstoffperoxid langsam versetzt. Das Reaktionsgemisch wird nun 48 Stunden bei Raumtemperatur weitergerührt, wobei das Reaktionsprodukt kristallin ausfällt. Letzteres wird abgenutscht, mit 50 ml Äthylmethylketon gewaschen und unter Vakuum bei 70 °C bis zum konstanten Gewicht getrocknet.

Man erhält 12 g (etwa 56% der Theorie) der Verbindung der Formel

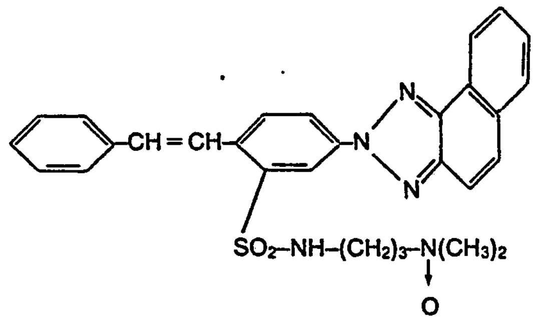

(602)

in Form eines gelben kristallinen Pulvers, das bei 157 bis 160 °C unter Zersetzung schmilzt. Nach einmaligem Umkristallisieren aus Alkohol erhält man 10 g der Verbindung der Formel (602) als blassgelbe Plättchen mit einem Schmelzpunkt von 165–170 °C (Zersetzung).

Die Herstellung der Ausgangsverbindung der Formel (601) ist in Beispiel 5 der britischen Patentschrift Nr. 917 242 beschrieben.

Beispiel 7:

22,5 g der Verbindung der Formel

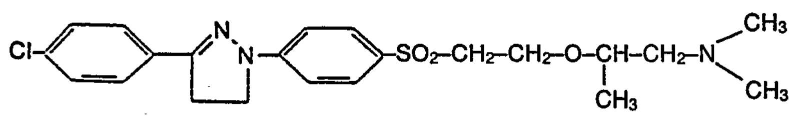

(701)

werden in 250 ml Äthylmethylketon gelöst und bei Raumtemperatur innerhalb 10 Minuten mit 57 g 30%igem Wasserstoffperoxid versetzt. Das Reaktionsgemisch wird 20 Stunden bei Raumtemperatur und 6 Stunden bei 40 °C weitergerührt, wobei das Reaktionsprodukt in Lösung bleibt.

Die erhaltene klare Reaktionslösung wird wie im Beispiel 3 beschrieben aufgearbeitet, wobei nach Einengen der wässrigen Reaktionslösung zur Trockene am Rotationsverdampfer unter Vakuum das Reaktionsprodukt aus Isopropanol unter Entfärbung mit 5 g Aktivkohle umkristallisiert wird.

Man erhält 17 g (etwa 73% der Theorie) der Verbindung der Formel

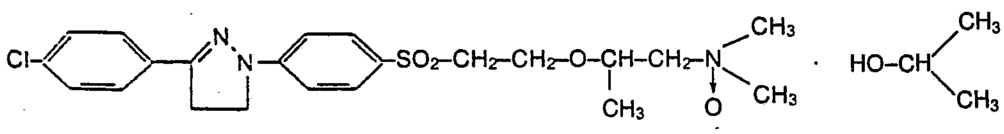

(702)

in Form von weissen Nädelchen, die bei 165 bis 170 °C unter Zersetzung schmelzen. Nach weiterem Umkristallisieren aus Isopropanol erhält man 10 g der Verbindung der Formel (702) in Form von weissen Nädelchen mit einem Schmelzpunkt von 170 bis 172 °C (Zersetzung).

Die Herstellung der Ausgangsverbindung der Formel (701) ist im Beispiel 1 der schweizerischen Patentschrift Nr. 452 886 beschrieben.

Beispiel 8:

22 g der Verbindung der Formel

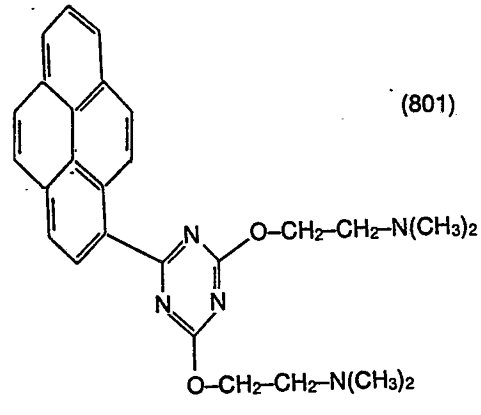

(801)

werden in 250 ml Äthylmethylketon heiss gelöst und die erhaltene klare Lösung wird bei Raumtemperatur innerhalb 10 Minuten mit 55 ml 30%igem Wasserstoffperoxid versetzt. Das Reaktionsgemisch wird 48 Stunden bei Raumtemperatur weitergerührt, wobei das Reaktionsprodukt langsam kristallin ausfällt. Letzteres wird abgenutscht, mit 50 ml Äthylmethylketon gewaschen und unter Vakuum bei 70 °C bis zum konstanten Gewicht getrocknet.

Man erhält 22 g (etwa 94% der Theorie) der Verbindung der Formel

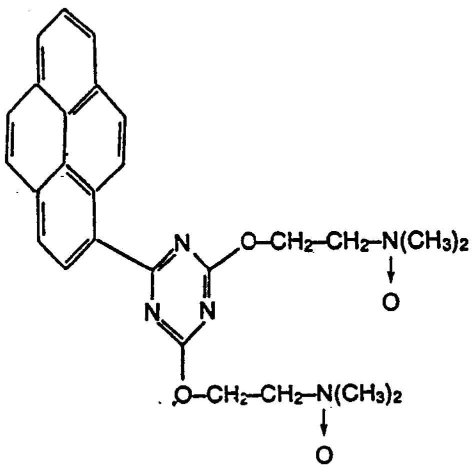

(802)

in Form eines gelben kristallinen Pulvers, das bei 152 °C unter Zersetzung schmilzt. Nach einmaligem Umkristallisieren aus Methanol-Isopropanol erhält man die Verbindung der Formel (802) in Form blassgelber Nädelchen mit einem Schmelzpunkt von 156–158 °C (Zersetzung).

Die als Ausgangsmaterial verwendete Verbindung der Formel (801) wird wie folgt hergestellt:

35 g der Verbindung der Formel

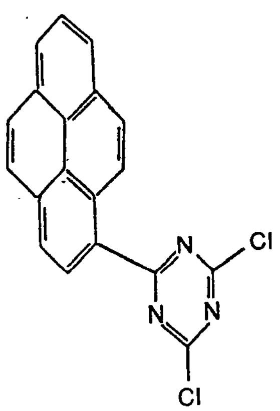

(803)

werden in 200 ml Methylenchlorid suspendiert und mit 4 g Benzyltributylammoniumbromid versetzt. Die erhaltene Suspension wird auf 0 °C gekühlt und bei dieser Temperatur zuerst mit einer Lösung von 26,8 g 2-Dimethylaminoäthanol in 50 ml Methylenchlorid innerhalb 10 Minuten und dann mit 200 ml einer 15%igen Natriumhydroxidlösung innerhalb 20 Minuten versetzt.

Das erhaltene zweiphasige Reaktionsgemisch wird zuerst eine Stunde bei 0 °C und dann 16 Stunden bei Raumtemperatur gerührt und dann mit 200 ml gesättigter Natriumchloridlösung verdünnt. Die Methylenchlorid-Schicht wird abdekantiert, mit gesättigter Natriumchlorid-Lösung neutral gewaschen, mit Natriumsulfat getrocknet und unter Vakuum vom Methylenchlorid befreit.

Man erhält 45 g (etwa 100% der Theorie) der Verbindung der Formel (801) in Form eines gelben Öls. Nach zweimaligem Umkristallisieren aus Essigester unter Zuhilfenahme von Aktivkohle erhält man die Verbindung der Formel (801) in Form von gelben Nädelchen mit einem Schmelzpunkt von 98–99 °C.

Die Herstellung der Ausgangsverbindung der Formel (803) ist in Beispiel 1 der britischen Patentschrift 985 484 beschrieben.

Beispiel 9:

Ein konzentriertes flüssiges Waschmittel wird durch Mischen folgender Komponenten hergestellt:

| | Gew.-% |
|---|---|
| Äthoxylierte Alkohole ($C_{12}$–$C_{13}$-Alkohol mit 6,5 Mol Äthylenoxid) | 60,0 |
| 1-Methyl-1-oleylamidoäthyl-2-oleylimidazolinium-methosulfat | 26,7 |
| Verbindung der Formel (102), (202) oder (204) | 0,3 |
| Wasser | 12,0 |
| Übliche Zusätze | 1,0 |

2 kg gebleichtes Baumwollgewebe werden während 10 Minuten bei 50 °C in 60 Litern Wasser von 100 ppm Härte gewaschen, die 50 bis 60 g des obigen Waschmittels enthalten. Nach dem Spülen und Trocknen weist das Gewebe einen starken Aufhelleffekt und einen weichen Griff auf.

Ähnliche Resultate werden erhalten, wenn anstelle des oben angegebenen, ein flüssiges Waschmittel folgender Zusammensetzung

| | Gew.-% |
|---|---|
| Äthoxylierte Alkohole ($C_{12}$–$C_{13}$-Alkohol mit 6,5 Mol Äthylenoxid) | 55,0 |
| 1-Methyl-1-talloylamidoäthyl-2-tallylimidazolinium-methosulfat | 26,0 |
| Verbindung der Formel (102), (202) oder (204) | 0,3 |
| Wasser | 13,0 |
| Isopropanol | 5,0 |
| übliche Zusätze | 0,7 |

oder ein anderes, nicht-ionische Tenside und kationische Substanzen enthaltendes flüssiges Waschmittel, wie z.B. das sich im Handel befindende «Perwoll® flüssig» oder Samtess®, verwendet wird, denen die erfindungsgemässen Aufheller der Formeln (102), (202) oder (204) beigemischt werden.

Ähnlich gute Resultate werden erhalten, wenn man den Waschvorgang mit einem der vorstehend genannten flüssigen Waschmittel wiederholt, das jedoch als Aufheller einen solchen der Formel (103), (104), (203), (302), (402), (502), (503), (602),

(702) oder (802) in der oben angegebenen Menge enthält.

Beispiel 10:

Ein flüssiges Waschmittel wird durch Mischen folgender Komponenten hergestellt:

| | Gew.-% |
|---|---|
| Äthoxylierte Alkohole ($C_{14}$–$C_{15}$-Alkohol mit 7 Mol Äthylenoxid) | 12,0 |
| Äthoxylierte Alkohole ($C_{12}$–$C_{13}$-Alkohol mit 6,5 Mol Äthylenoxid) | 12,0 |
| Nicht-gehärtetes Di-tallyl-dimethyl-ammoniumchlorid | 6,4 |
| Äthanol | 15,0 |
| Natriumbicarbonat | 0,25 |
| Verbindung der Formel (102), (202) oder (204) | 0,41 |
| Übliche Zusätze | 0,41 |
| Wasser | 53,53 |

Wie in Beispiel 9 beschrieben, wird gebleichtes Baumwollgewebe in einer Flotte gewaschen, die das vorstehend beschriebene Waschmittel enthält. Man erhält auf diese Weise stark aufgehelltes Baumwollgewebe mit einem weichen Griff.

Ähnlich gute Resultate werden erhalten, wenn man den Waschvorgang mit dem vorstehend genannten flüssigen Waschmittel wiederholt, das jedoch als Aufheller einen solchen der Formel (103), (104), (203), (302), (402), (502), (503), (602), (702) oder (802) in der oben angegebenen Menge enthält.

Beispiel 11:

Ein gebleichtes Baumwoll-Gewebe wird auf einem Färbeapparat bei einem Flottenverhältnis von 1:20 mit einem wässrigen Bad behandelt, das 0,1% eines Aufhellers der Formel (102), (103), (104), (202), (203) oder (204), bezogen auf das Gewicht der Baumwolle, und 5 g/l Natriumsulfat enthält.

Die Applikation erfolgt gemäss folgendem Temperaturprogramm:

20–50 °C: 15 Minuten
50 °C: 15 Minuten

Anschliessend wird das Baumwoll-Gewebe während 20 Sekunden in fliessendem enthärtetem Wasser gespült und bei 70 °C im Trockenschrank getrocknet. Das so behandelte Baumwoll-Gewebe weist einen guten Aufhelleffekt auf.

Beispiel 12:

Man foulardiert bei Raumtemperatur ein gebleichtes Baumwoll-Gewebe mit einer wässrigen Flotte, die 1 g/l eines Aufhellers der Formel (102), (103), (104), (202), (203), (204), (602), (702), (302), (402) oder (502) enthält. Der Abquetscheffekt beträgt 75%.

Anschliessend wird während 30 Sekunden bei 70 °C auf einem Thermofixiergerät getrocknet.

Das so behandelte Baumwollgewebe weist einen guten Aufhelleffekt auf.

Beispiel 13:

Ein Polyacrylnitril-Gewebe (®Orlon 75) wird auf einem Färbeapparat bei einem Flottenverhältnis von 1:20 mit einem wässrigen Bad, das 0,1% eines Aufhellers der Formel (102), (202), (203), (204), (302), (402), (502), (503) oder (702), bezogen auf das Gewicht des Gewebes, 1 g/l eines Anlagerungsproduktes von 35 Mol Äthylenoxid an 1 Mol Stearylalkohol und 1,5 ml/l Ameisensäure 85% enthält, behandelt.

Die Applikation erfolgt gemäss folgendem Temperaturprogramm:

40–97 °C: 30 Minuten
97 °C: 30 Minuten
97–40 °C: 15 Minuten

Anschliessend wird das Polyacrylnitril-Gewebe während 20 Sekunden in fliessendem enthärtetem Wasser gespült und bei 70 °C im Trockenschrank getrocknet. Das so behandelte Gewebe weist einen guten Aufhelleffekt auf.

Beispiel 14:

Ein gebleichtes Baumwoll-Gewebe wird im Flottenverhältnis 1:20 während 15 Minuten in einer 30 °C warmen, wässrigen Weichspülflotte behandelt, die pro Liter 0,2 g Dimethyldistearyl-ammoniumchlorid und 0,01 g eines Aufhellers der Formel (102), (103), (104), (202), (203), (204) oder (602) enthält.

Anschliessend wird das Baumwoll-Gewebe während 5 Sekunden in fliessendem Wasser gespült und bei 70 °C im Trockenschrank getrocknet. Da so behandelte Baumwoll-Gewebe weist einen guten Aufhelleffekt auf.

Beispiel 15:

Ein gebleichtes Baumwoll-Gewebe wird im Flottenverhältnis 1:20 während 15 Minuten in einer 40 °C warmen, wässrigen Flotte gewaschen, die pro Liter 0,5 g eines Anlagerungsproduktes von 10 Mol Äthylenoxid an ein Mol Stearylalkohol und 0,01 g eines Aufhellers der Formel (102), (103), (104), (202), (203), (204) oder (802) enthält.

Anschliessend wird das Baumwoll-Gewebe während 20 Sekunden in fliessendem Wasser gespült und bei 70 °C im Trockenschrank getrocknet. Das so behandelte Baumwoll-Gewebe weist einen guten Aufhelleffekt auf.

Beispiel 16:

Ein modifiziertes Polyacrylnitrilgewebe (Courtelle®) wird auf einem Färbeapparat bei einem Flottenverhältnis von 1:20 mit einem wässerigen Bad, das 0,1% eines Aufhellers der Formel (102), (202), (203), (204), (302), (402), (502), (503) oder (702), bezogen auf das Gewicht des Gewebes, 1 g/l Oxalsäure, 0,25 g/l eines Polyphosphates als Komplexbildner und 0,125 g/l Natriummetabisulfit enthält, behandelt. Die Applikation erfolgt gemäss folgendem Temperaturprogramm:

40–97 °C: 30 Minuten
97 °C: 30 Minuten
97–40 °C: 15 Minuten

Anschliessend wird das Polyacrylnitril-Gewebe während 30 Sekunden in fliessendem enthärtetem Wasser gespült und bei 70 °C im Trockenschrank

getrocknet. Das so behandelte Gewebe weist einen guten Aufhelleffekt auf.

Beispiel 17:

Ein Polyamid-6-Gewebe wird auf einem Färbeapparat bei einem Flottenverhältnis von 1:20 mit einem wässrigen Bad behandelt, das 0,3%, bezogen auf das Gewebegewicht, eines Aufhellers der Formel (102), (103), (104), (202), (203), (204), (302), (602) oder (802), 1 g/l eines Anlagerungsproduktes von 35 Mol Äthylenoxid an 1 Mol Stearylalkohol, 1 g/l eines Anlagerungsproduktes von 8 Mol Äthylenoxid an 1 Mol p-tert. Octylphenol und 0,5 g/l Natriumphosphatpuffer enthält. Die Applikation erfolgt nach folgendem Temperaturprogramm:

50–100 °C innerhalb 10 Minuten,
100 °C während 20 Minuten
100–50 °C innerhalb 5 Minuten.

Anschliessend wird das Gewebe in enthärtetem kaltem Wasser gespült und bei 60 °C getrocknet. Das so behandelte Gewebe weist einen guten Aufhelleffekt auf.

Beispiel 18:

Ein Polyamid-6-Gewebe wird auf einem Färbeapparat bei einem Flottenverhältnis von 1:20 mit einem wässrigen Bad behandelt, das 0,3%, bezogen auf das Gewebegewicht, eines Aufhellers der Formel (102), (103), (202), (204), (302) oder (602), 1 g/l eines Anlagerungsproduktes von 35 Mol Äthylenoxid an 1 Mol Stearylalkohol, 1 g/l eines Anlagerungsproduktes von 8 Mol Äthylenoxid an 1 Mol p-tert. Octylphenol und 0,5 g/l Natriumphosphatpuffer enthält. Die Applikation erfolgt gemäss folgendem Temperaturprogramm:

30–60 °C innerhalb 10 Minuten,
60 °C während 20 Minuten.

Anschliessend wird das Gewebe in enthärtetem kaltem Wasser gespült und bei 60 °C getrocknet. Das so behandelte Gewebe weist einen guten Aufhelleffekt auf.

Beispiel 19:

5 g Faserstoff (bestehend aus gebleichter Sulfitzellulose und gebleichter Buchenzellulose 1:1) in 50 ml Wasser werden in einem Mixer mit 150 ml Aufhellerlösung, enthaltend 2,5 mg (entsprechend einer Konzentration von 0,05%) eines Aufhellers der Formel (702) oder (802) während 15 Minuten gemischt. Anschliessend werden 1,5 Gew.-% Leim, z.B. Bewoidleim® und 2,5 Gew.% Aluminiumsulfat (bezogen auf das Fasertrockengewicht) zugegeben und mit Wasser von ca. 10° dH auf 1000 ml verdünnt. Aus dieser Fasersuspension wird ein Papierblatt hergestellt, das einen guten Aufhelleffekt aufweist.

Beispiel 20:

5 g Faserstoff (bestehend aus gebleichter Sulfitzellulose und gebleichter Buchenzellulose 1:1) in 150 ml Wasser, enthaltend 5 mg eines kationaktiven Polyätheramins, werden in einem Mixer mit 50 ml Aufhellerlösung, enthaltend 2,5 mg (entsprechend einer Konzentration von 0,05%) eines Aufhellers der Formel (502) oder (802) während 15 Minuten gemischt. Anschliessend werden 1,5 Gew.% Leim, z.B. Bewoidleim®, 2,5 Gew.% Aluminiumsulfat und 0,1% eines kationaktiven Polyätheramins (bezogen auf das Fasertrockengewicht) zugegeben und mit Wasser von ca. 10° dH auf 1000 ml verdünnt. Aus dieser Fasersuspension wird ein Papierblatt hergestellt, das einen guten Aufhelleffekt aufweist.

Beispiel 21:

5 g Faserstoff (bestehend aus gebleichter Sulfitzellulose und gebleichter Buchenzellulose 1:1) in 150 ml Wasser, enthaltend 5 mg eines Polyäthylenimins, werden in einem Mixer mit 50 ml Aufhellerlösung, enthaltend 2,5 mg (entsprechend einer Konzentration von 0,05%) eines Aufhellers der Formel (702) oder (802) während 15 Minuten gemischt. Anschliessend werden 1,5 Gew.% Leim, z.B. Bewoidleim®, 2,5 Gew.% Aluminiumsulfat und 0,1% eines Polyäthylenimins (bezogen auf das Fasertrockengewicht) zugegeben und mit Wasser von ca. 10° dH auf 1000 ml verdünnt. Aus dieser Fasersuspension wird ein Papierblatt hergestellt, das einen guten Aufhelleffekt aufweist.

Beispiel 22:

Eine innige Mischung aus 100 Teilen Polyvinylchlorid, 3 Teilen Stabilisator (Advastab BD 100 ®; Ba/Cd-Komplex), 2 Teilen Titandioxid, 59 Teilen Dioctylphthalat und 0,01 bis 0,2 Teilen eines Aufhellers der Formel (802) wird auf einem Kalander bei 150 bis 155 °C zu einer Folie ausgewalzt. Die so gewonnene opake Polyvinylchloridfolie besitzt einen wesentlich höheren Weissgehalt als eine Folie, welche den optischen Aufheller nicht enthält.

**Patentansprüche**

1. Aminoxidverbindungen der Formel

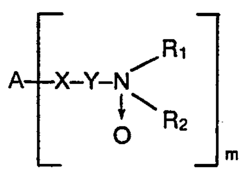

worin

m die Zahl 1 oder 2,

A den Rest eines Aufhellersystems aus der Gruppe der 2-Furanyl-benzimidazole, 2-Azolylbenzimidazole, 2-Stilbenyl-benzoxazole, 2-Stilbenyl-benzimidazole, 1,2-Bis-azolyläthylene, 2,5-Bis-benzimidazolylfurane, 4,4'-Bis-azolylstilbene, 2-Phenyl-5-azolylthiophene, 1,3-Diphenylpyrazoline, 4,4'-Distyrylbiphenyle, 4,4'-Divinyl-stilbene, 1,4-Distyrylbenzole, 3,7-disubstituierten Cumarine, Naphthalimide, 2-Stilben-4-yl-naphthotriazole, 4,4'-Bis-triazolyl- oder -pyrazolyl-stilbene, Triazolyl- oder Pyrazolyl-stilbene, Naphthotriazolylstilbene, Triazinylpyrene oder Bis-styryl-dibenzofurane, wobei diese Reste unsubstituiert oder durch Halogen, Alkyl, Hydroxyalkyl, Halogenalkyl, Cyanoalkyl, Alkoxyalkyl, Phenylalkyl, Carboxyalkyl, Carbalkoxyalkyl, Alkenyl, Cycloalkyl, Alkoxy,

Alkenyloxy, Alkoxycarbonyl, Aminocarbonyl, Alkyl- oder Dialkylaminocarbonyl, Cyano, Alkylsulfonyl, Alkoxysulfonyl, Aminosulfonyl, Hydroxy, Carboxy, Sulfo oder Trifluormethyl substituiert sind,

X eine direkte Bindung zwischen A und Y, ein Sauerstoff- oder Schwefelatom oder eine Gruppe der Formel $-SO_2-$, $-SO_2-O-$, $-COO-$, $-CON(R)-$ oder $-SO_2N(R)-$, worin R für Wasserstoff oder unsubstituiertes oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituiertes Alkyl steht,

Y eine unsubstituierte oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituierte geradkettige oder verzweigte Alkylen- oder Alkylenoxyalkylengruppe,

$R_1$ und $R_2$ unabhängig voneinander Cyclohexyl, unsubstituiertes oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituiertes Alkyl, Phenyl oder $R_1$ und $R_2$ zusammen mit dem Stickstoffatom, an das sie gebunden sind, einen 5- oder 6-gliedrigen gesättigten oder ungesättigten Heterocyclus, der gegebenenfalls noch ein oder zwei weitere Heteroatome als Ringglieder enthalten kann, wobei gegebenenfalls ein gesättigter Heterocyclus durch 1 oder 2 $C_1$–$C_4$-Alkylgruppen und ein ungesättigter Heterocyclus durch Halogen, Alkyl oder Alkoxy substituiert sein kann, bedeuten;

oder worin für den Fall, dass X für eine Gruppe der Formel $-CON(R)-$ oder $-SO_2N(R)-$ und Y für Äthylen oder Propylen stehen, R und $R_1$ gemeinsam die Äthylen- oder Methylengruppe bedeuten, wodurch ein entsprechender, 2 Stickstoffatome enthaltender, gesättigter Heterocyclus entsteht.

2. Aminoxidverbindungen nach Anspruch 1, worin X ein Sauerstoff- oder Schwefelatom oder eine Gruppe der Formel $-SO_2-$, $-CON(R)-$ oder $-SO_2N(R)-$ und Y eine geradkettige oder verzweigte Alkylengruppe mit 1 bis 12 C-Atomen bedeuten, wobei R wie in Anspruch 1 definiert ist und R und $R_1$ gegebenenfalls zusammen die in Anspruch 1 angegebene Bedeutung haben können.

3. Aminoxidverbindungen nach Anspruch 1 der Formel

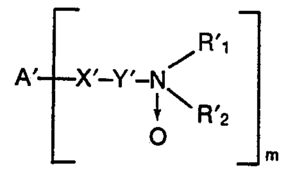 ,

worin

A′ einen unsubstituierten oder durch Halogen, Alkyl, Hydroxyalkyl, Halogenalkyl, Cyanoalkyl, Alkoxyalkyl, Phenylalkyl, Carboxyalkyl, Carbalkoxyalkyl, Alkenyl, Cycloalkyl, Alkoxy, Alkenyloxy, Alkoxycarbonyl, Aminocarbonyl, Alkyl- oder Dialkylaminocarbonyl, Cyano, Alkylsulfonyl, Alkoxysulfonyl, Aminosulfonyl, Hydroxy, Carboxy, Sulfo oder Trifluormethyl substituierten Rest der Formeln

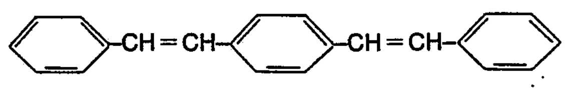 ,

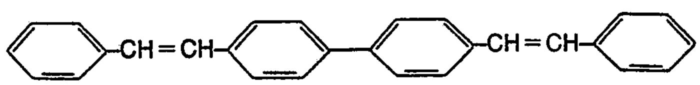 ,

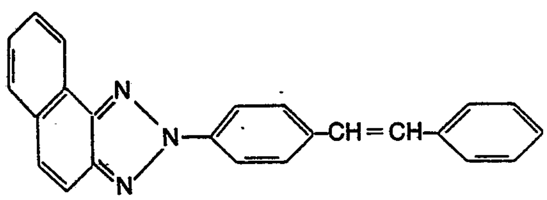 ,

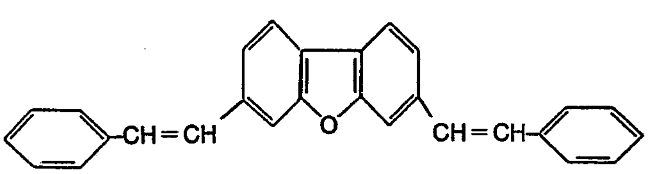 ,

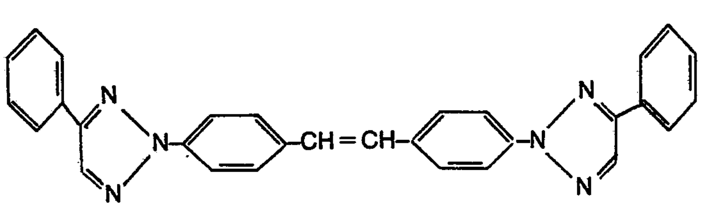 ,

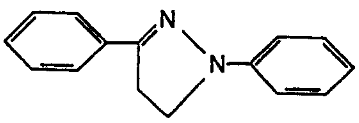

oder

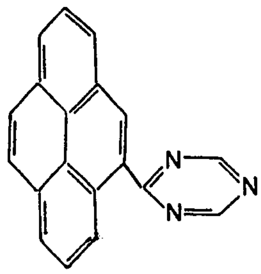

,

X′ ein Sauerstoffatom oder eine Gruppe der Formel $-SO_2N(R')-$ oder $-SO_2-$, wobei

R′ für Wasserstoff oder unsubstituiertes oder mit Hydroxy, Cyano oder Halogen substituiertes Alkyl mit 1 bis 4 C-Atomen im Alkylteil steht,

Y′ eine geradkettige oder verzweigte Alkylen- oder Alkylenoxyalkylengruppe,

m die Zahl 1 oder 2 und

$R_1'$ und $R_2'$ unabhängig voneinander unsubstituiertes oder mit Halogen, Cyano, Hydroxy, Alkoxy mit 1 bis 4 C-Atomen, Phenyl, Chlorphenyl, Methoxyphenyl, Methylphenyl oder Alkoxycarbonyl mit 2 bis 5 C-Atomen substituiertes Alkyl mit 1 bis 8 C-Atomen im Alkylteil oder $R_1'$ und $R_2'$ zusammen mit dem Stickstoffatom, an das sie gebunden sind, einen 5- oder 6-gliedrigen gesättigten oder ungesättigten Heterocyclus, der gegebenenfalls noch ein oder zwei weitere Heteroatome als Ringglieder enthalten kann, wobei ein gegebenenfalls gesättigter Heterocyclus durch 1 oder 2 $C_1$–$C_4$-Alkylgruppen und ein ungesättigter Heterocyclus durch Halogen, Alkyl oder Alkoxy substituiert sein kann, bedeuten; oder worin für den Fall, dass X′ für eine Gruppe der Formel $-SO_2N(R')-$ und Y′ für Äthylen oder Propylen stehen, R′ und $R_1'$ gemeinsam die Äthylen- oder Methylengruppe bedeuten, wodurch eine Gruppierung der Formel

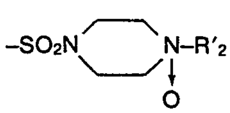

,

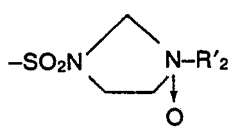

oder

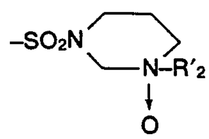

entsteht.

4. Aminoxidverbindungen nach Anspruch 3 der Formel

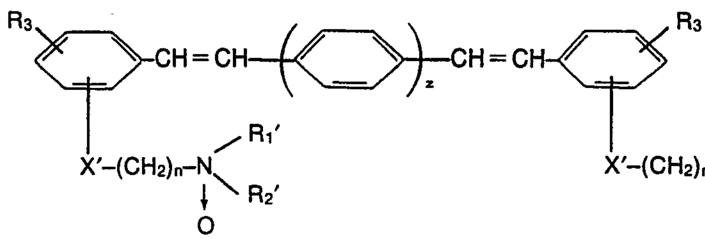

worin z für die Zahl 1 oder 2 und n für eine ganze Zahl zwischen 1 und 4 stehen, X′, $R_1'$ und $R_2'$ wie in Anspruch 3 definiert sind und $R_3$ Halogen, Alkyl mit 1 bis 4 C-Atomen, Cycloalkyl mit 5 oder 6 C-Atomen, Alkoxy mit 1 bis 4 C-Atomen, Alkylsulfonyl mit 1 bis 4 C-Atomen, Phenylsulfonyl, Carbalkoxy mit 2 bis 5 C-Atomen, Carbamoyl oder Sulfamoyl bedeutet, insbesondere Verbindungen der Formel

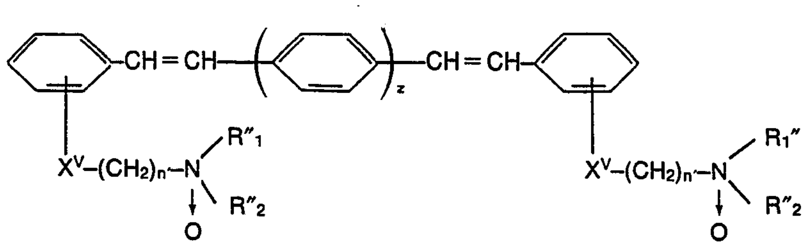

,

worin

z die Zahl 1 oder 2,

$X^V$ ein Sauerstoffatom oder eine Gruppe der Formel $-SO_2N(R')-$, wobei R′ für Wasserstoff oder unsubstituiertes oder mit Hydroxy, Cyano oder Halogen substituiertes Alkyl mit 1 bis 4 C-Atomen im Alkylteil steht,

n′ eine ganze Zahl zwischen 1 und 3 und

$R_1''$ und $R_2''$ unabhängig voneinander Alkyl oder Hydroxyalkyl mit 1 bis 4 C-Atomen bedeuten, sowie der Formel

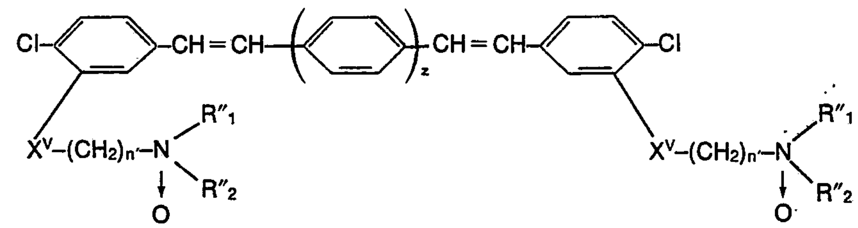 ,

worin

z die Zahl 1 oder 2,

$X^v$ ein Sauerstoffatom oder eine Gruppe der Formel $-SO_2N(R')-$, wobei R′ für Wasserstoff oder unsubstituiertes oder mit Hydroxy, Cyano oder Halogen substituiertes Alkyl mit 1 bis 4 C-Atomen im Alkylteil steht,

n′ eine ganze Zahl zwischen 1 und 3 und

$R_1''$ und $R_2''$ unabhängig voneinander Alkyl oder Hydroxyalkyl mit 1 bis 4 C-Atomen bedeuten.

5. Aminoxidverbindungen nach Anspruch 3 der Formel

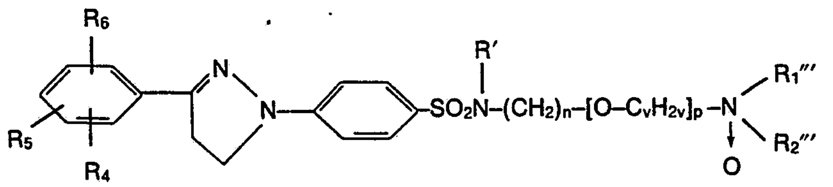 ,

worin $R_4$ Wasserstoff oder Halogen, $R_5$ und $R_6$ jeweils Wasserstoff, Halogen, Alkyl oder Alkoxy mit je 1 bis 4 C-Atomen, n und v jeweils eine ganze Zahl zwischen 1 und 4, p 0 oder 1, R′ Wasserstoff, unsubstituiertes oder mit Hydroxy, Cyano oder Halogen substituiertes Alkyl mit 1 bis 4 C-Atomen im Alkylteil,

$R_1'''$ und $R_2'''$ unabhängig voneinander unsubstituiertes oder mit Halogen, Cyano, Hydroxy, Alkoxy mit 1 bis 4 C-Atomen, Phenyl, Chlorphenyl, Methoxyphenyl, Methylphenyl oder Alkoxycarbonyl mit 2 bis 5 C-Atomen substituiertes Alkyl mit 1 bis 8 C-Atomen im Alkylteil bedeuten oder worin für den Fall, dass n für 2 oder 3 und p für 0 steht, R′ und $R_1'''$ gemeinsam die Äthylen- oder Methylengruppe bedeuten, wodurch eine Gruppierung der Formel

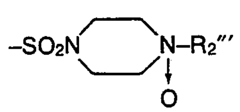 , 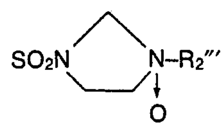 oder 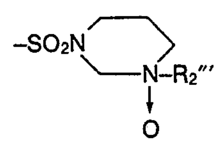

entsteht, insbesondere Verbindungen der Formel

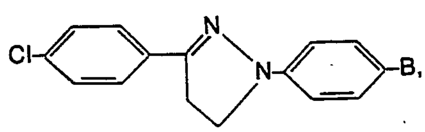

worin B eine Gruppe der Formel

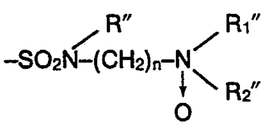 , 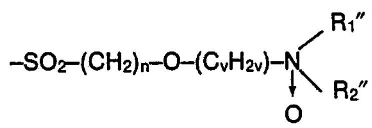 oder 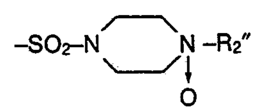

bedeutet, worin R″ für Wasserstoff, Alkyl, Hydroxyalkyl oder Cyanoalkyl mit jeweils 1 bis 4 C-Atomen im Alkylteil, $R_1''$ und $R_2''$ jeweils für Alkyl oder Hydroxyalkyl mit 1 bis 4 C-Atomen und n und v jeweils für eine ganze Zahl zwischen 1 und 4 stehen.

6. Aminoxidverbindungen nach Anspruch 3 der Formel

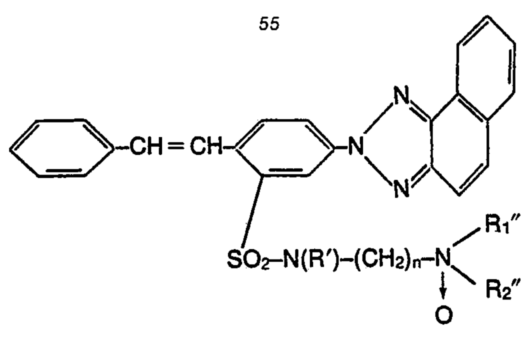 ,

worin R' Wasserstoff, Alkyl, Hydroxyalkyl, Cyanoalkyl oder Halogenalkyl mit jeweils 1 bis 4 C-Atomen im Alkylteil, $R_1''$ und $R_2''$ jeweils Alkyl oder Hydroxyalkyl mit 1 bis 4 Kohlenstoffatomen und n eine ganze Zahl zwischen 1 und 4 bedeuten.

7. Aminoxidverbindungen nach Anspruch 3 der Formel

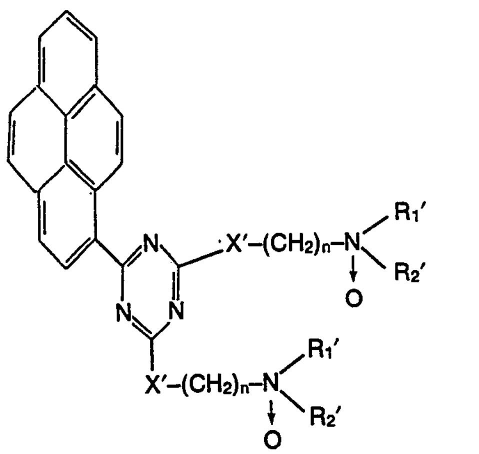
,

worin n für eine ganze Zahl zwischen 1 und 4 steht und X', $R_1'$ und $R_2'$ wie in Anspruch 3 definiert sind, wobei X' vorzugsweise Sauerstoff und $R_1'$ und $R_2'$ vorzugsweise unabhängig voneinander Alkyl oder Hydroxyalkyl mit 1 bis 4 C-Atomen bedeuten.

8. Verfahren zur Herstellung von Aminoxidverbindungen der im Anspruch 1 definierten Formel, worin X eine direkte Bindung zwischen A und Y, ein Sauerstoffatom oder eine Gruppe der Formel $-SO_2-O-$, $-SO_2-$, $-COO-$, $-CON(R)-$ oder $-SO_2N(R)-$ bedeutet, worin R für Wasserstoff oder unsubstituiertes oder durch Halogen, Hydroxy, $C_2$–$C_5$-Carbalkoxy, $C_1$–$C_4$-Alkoxy, Phenyl, Chlorphenyl, Methylphenyl, Methoxyphenyl, Carbamoyl oder Sulfamoyl substituiertes Alkyl steht, dadurch gekennzeichnet, dass man eine Verbindung der Formel

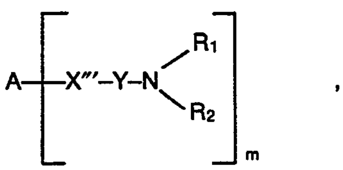
,

worin A, Y, $R_1$, $R_2$ und m wie in Anspruch 1 definiert sind und X''' eine direkte Bindung zwischen A und Y, ein Sauerstoff- oder Schwefelatom oder eine Gruppe der Formel $-SO-$, $-SO_2-$, $-SO_2-O-$, $-COO-$, $-CON(R)-$ oder $-SO_2N(R)-$ bedeutet, wobei R wie oben definiert ist, oxidiert, vorzugsweise mit einer «Per-Verbindung», die in äquivalenter Menge oder im Überschuss eingesetzt wird, insbesondere mit Perameisensäure, Peressigsäure oder Wasserstoffperoxid oder Gemischen dieser Per-Verbindungen, vor allem aber mit Wasserstoffperoxid.

9. Verfahren zur Herstellung von Aminoxidverbindungen der im Anspruch 1 definierten Formel, worin X ein Sauerstoff- oder Schwefelatom oder eine Gruppe der Formel $-SO_2-$ oder $-COO-$ bedeutet, dadurch gekennzeichnet, dass man ein Moläquivalent einer Verbindung der Formel

$$A-[-X''-H]_m$$

mit m Moläquivalent einer Verbindung der Formel

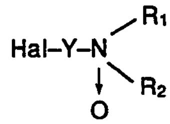

umsetzt, in welchen Formeln A, m, Y, $R_1$ und $R_2$ wie in Anspruch 1 definiert sind, Hal für Chlor oder Brom und X'' für ein Sauerstoff- oder Schwefelatom oder eine Gruppe der Formel $-SO_2-$ oder $-COO-$ stehen.

10. Verfahren zur Herstellung von Aminoxidverbindungen der im Anspruch 1 definierten Formel, worin X eine Gruppe der Formel $-COO-$ oder $-SO_2-O-$ bedeutet, dadurch gekennzeichnet, dass man ein Moläquivalent einer Verbindung der Formel

$$A-[-SO_2Hal]_m \quad \text{oder} \quad A-[-COHal]_m$$

mit m Moläquivalent einer Verbindung der Formel

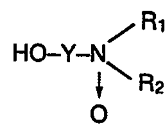

umsetzt, in welchen Formeln A, m, Y, $R_1$ und $R_2$ wie in Anspruch 1 definiert sind und Hal für Chlor oder Brom steht.

11. Verwendung von Aminoxidverbindungen der im Anspruch 1 angegebenen Formel als optische Aufheller für hochmolekulare organische Materialien, insbesondere für Materialien aus Polyacrylnitril, Polyamid und Cellulose, vorzugsweise als optische Aufheller im Waschbad.

12. Waschmittel, enthaltend eine oder mehrere Aminoxidverbindungen der im Anspruch 1 definierten Formel.

13. Waschmittel nach Anspruch 12, welches 10 bis 70 Gew.% eines nichtionogenen Tensids und 1 bis 30 Gew.% eines kationischen Textilweichmachers, wobei letzterer vorzugsweise ein quaternäres Derivat des Ammoniaks oder des Imidazolins mit 2 langkettigen, aliphatischen gesättigten oder ungesättigten Resten oder eine Mischung aus beiden ist, und welches Waschmittel gegebenenfalls, zur Verleihung der flüssigen Form, noch ein Lösungsmittel enthält.

14. Waschmittel nach Anspruch 13, welches 1-Methyl-1-oleylamidoäthyl-2-oleyl-imidazolinium · $X^\ominus$, 1-Methyl-1-talloylamidoäthyl-2-tallylimidazolinium · $X^\ominus$, Di-tallyl-dimethyl-ammonium · $X^\ominus$ oder eine Verbindung der Formel

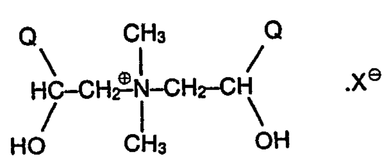
,

worin Q $C_{14-16}$-Alkyl bedeutet und $X^{\ominus}$ für ein Chlorid-, Bromid-, Methylsulfat-, Äthylsulfat-, Methan-, Äthan- oder Toluolsulfonatanion steht, als kationischen Textilweichmacher enthält.

15. Textilbehandlungsmittel oder Wäschenachbehandlungsmittel, enthaltend eine oder mehrere Aminoxidverbindungen der im Anspruch 1 definierten Formel und ein kationisches Textilbehandlungsmittel oder einen kationischen Textilweichmacher.

## Claims

1. An amine oxide compound of the formula

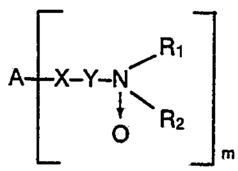

in which m is the integer 1 or 2, A is the radical of a brightener system from the group of the 2-furanyl-benzimidazoles, 2-azolyl-benzimidazoles, 2-stilbenyl-benzoxazoles, 2-stilbenyl-benzimidazoles, 1,2-bis-azolylethylenes, 2,5-bis-benzimidazolylfurans, 4,4′-bis-azolylstilbenes, 2-phenyl-5-azolyl-thiophenes, 1,3-diphenylpyrazolines, 4,4′-distyryl-biphenyls, 4,4′-divinyl-stilbenes, 1,4-distyrylbenzenes, 3,7-disubstituted coumarins, naphthalimides, 2-stilben-4-yl-naphthotriazoles, 4,4′-bis-triazolyl-stilbenes, 4,4′-bis-pyrazolyl-stilbenes, triazolyl-stilbenes, pyrazolyl-stilbenes, naphthotriazolyl-stilbenes, triazinyl-pyrenes and bis-styryl-dibenzofurans, these radicals being monosubstituted or substituted by halogen, alkyl, hydroxyalkyl, halogenoalkyl, cyanoalkyl, alkoxyalkyl, phenylalkyl, carboxyalkyl, carbalkoxyalkyl, alkenyl, cycloalkyl, alkoxy, alkenyloxy, alkoxycarbonyl, aminocarbonyl, alkylaminocarbonyl, dialkylaminocarbonyl, cyano, alkylsulfonyl, alkoxysulfonyl, aminosulfonyl, hydroxyl, carboxyl, sulfo and trifluoromethyl, X is a direct bond between A and Y, an oxygen atom or sulfur atom or a group of the formula $-SO_2-$, $-SO_2-O-$, $-COO-$, $-CON(R)-$ or $-SO_2N(R)-$, in which R is hydrogen or alkyl which is unsubstituted or substituted by halogen, hydroxyl, $C_2$–$C_5$carbalkoxy, $C_1$–$C_4$alkoxy, phenyl, chlorophenyl, methylphenyl, methoxyphenyl, carbamoyl or sulfamoyl, Y is a straight chain or branched alkylene or alkyleneoxyalkylene which is unsubstituted or substituted by halogen, hydroxyl, $C_2$–$C_5$carbalkoxy, $C_1$–$C_4$alkoxy, phenyl, chlorophenyl, methylphenyl, methoxyphenyl, carbamoyl or sulfamoyl, and $R_1$ and $R_2$ independently of one another are cyclohexyl, phenyl or alkyl which is unsubstituted or substituted by halogen, hydroxyl, $C_2$–$C_5$carbalkoxy, $C_1$–$C_4$alkoxy, phenyl, chlorophenyl, methylphenyl, methoxyphenyl, carbamoyl or sulfamoyl, or $R_1$ and $R_2$, together with the nitrogen atom to which they are bonded, are a 5-membered or 6-membered saturated or unsaturated heterocyclic ring which can additionally contain one or two other hetero atoms as ring members, where a saturated heterocyclic ring may be substituted by 1 or 2 $C_1$–$C_4$alkyl groups and an unsaturated heterocyclic ring may be substituted by halogen, alkyl or alkoxy; and in which, if X is a group of the formula $-CON(R)-$ or $-SO_2N(R)-$ and Y is ethylene or propylene, R and $R_1$ together are an ethylene or methylene group, thereby forming a corresponding saturated heterocyclic ring containing 2 nitrogen atoms.

2. An amine oxide compound according to claim 1, in which X is an oxygen atom or sulfur atom or a group of the formula $-SO_2-$, $-CON(R)-$ or $-SO_2N(R)-$ and Y is a straight-chain or branched alkylene group having 1 to 12 carbon atoms, R being as defined in claim 1 or R and $R_1$ together being as defined in claim 1.

3. An amine oxide compound according to claim 1, of the formula

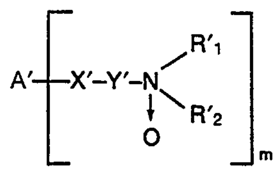

in which A′ is a radical of the formula

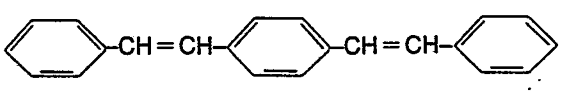 ,

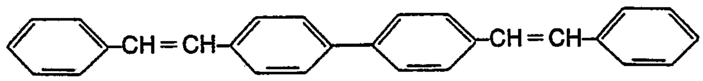 ,

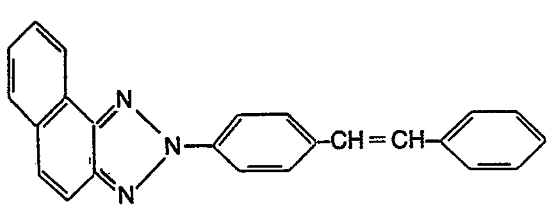 ,

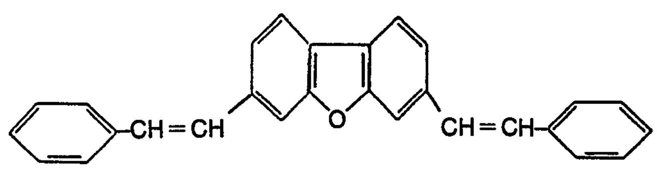

,

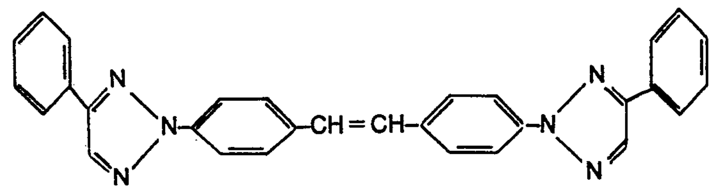

,

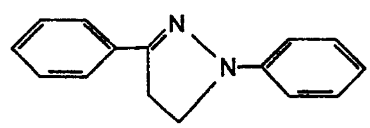

or ,

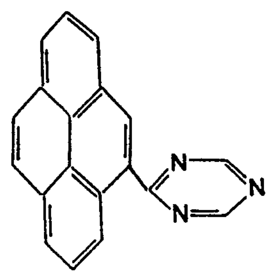

,

which is unsubstituted or substituted by halogen, alkyl, hydroxyalkyl, halogenoalkyl, cyanoalkyl, alkoxyalkyl, phenylalkyl, carboxyalkyl, carbalkoxyalkyl, alkenyl, cycloalkyl, alkoxy, alkenyloxy, alkoxycarbonyl, aminocarbonyl, alkylaminocarbonyl, dialkylaminocarbonyl, cyano, alkylsulfonyl, alkoxysulfonyl, aminosulfonyl, hydroxyl, carboxyl, sulfo or trifluoromethyl, X′ is an oxygen atom or a group of the formula $-SO_2N(R')-$ or $-SO_2-$, R′ being hydrogen or alkyl, which has 1 to 4 carbon atoms and is unsubstituted or substituted by hydroxyl, cyano or halogen, Y′ is a straight-chain or branched alkylene or alkyleneoxyalkylene group, m is the integer 1 or 2 and $R_1'$ and $R_2'$ independently of one another are alkyl which has 1 to 8 carbon atoms, and is unsubstituted or substituted by halogen, cyano, hydroxyl, alkoxy having 1 to 4 carbon atoms, phenyl, chlorophenyl, methoxyphenyl, methylphenyl or alkoxycarbonyl having 2 to 5 carbon atoms, or $R_1'$ and $R_2'$ together with the nitrogen atom to which they are bonded are a 5-membered or 6-membered saturated or unsaturated heterocyclic ring which can contain one or two further hetero atoms as ring members, where a saturated heterocyclic ring may be substituted by 1 or 2 $C_1$–$C_4$alkyl groups and an unsaturated heterocyclic ring may be substituted by halogen, alkyl or alkoxy; or in which, if X′ is a group of the formula $-SO_2N(R')-$ and Y′ is ethylene or propylene, $R_1'$ and $R_2'$ conjointly are an ethylene or methylene group, thereby forming a group of the formula

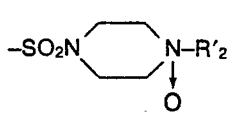

,

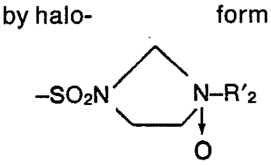

or

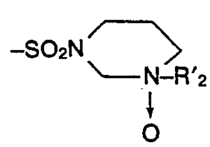

4. An amine oxide compound according to claim 3, of the formula

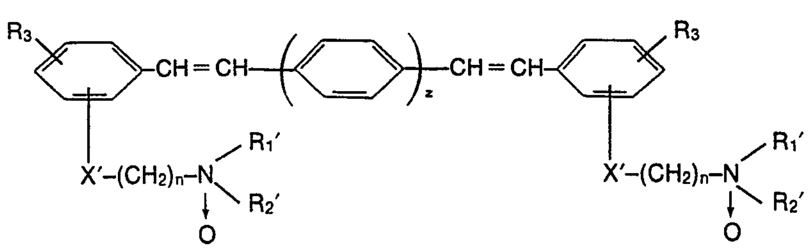

in which z is the integer 1 or 2 and n is an integer between 1 and 4, $R_1'$ and $R_2'$ are defined as in claim 3 and $R_3$ is halogen, alkyl having 1 to 4 carbon atoms, cycloalkyl having 5 or 6 carbon atoms, alkoxy having 1 to 4 carbon atoms, alkylsulfonyl having 1 to 4 carbon atoms, phenylsulfonyl, carbalkoxy having 2 to 5 carbon atoms, carbamoyl or sulfamoyl, in particular a compound of the formula

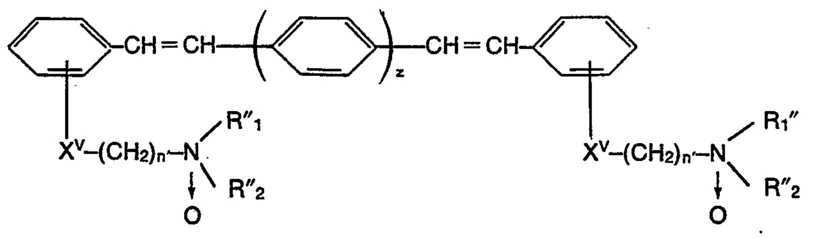

,

in which z is the integer 1 or 2, $X^V$ is an oxygen atom or a group of the formula $-SO_2N(R')-$, R' being hydrogen or alkyl which has 1 to 4 carbon atoms and is unsubstituted or substituted by hydroxyl, cyano or halogen, n' is an integer between 1 and 3 and $R_1''$ and $R_2''$ independently of one another are alkyl or hydroxyalkyl having 1 to 4 carbon atoms, and of the formula

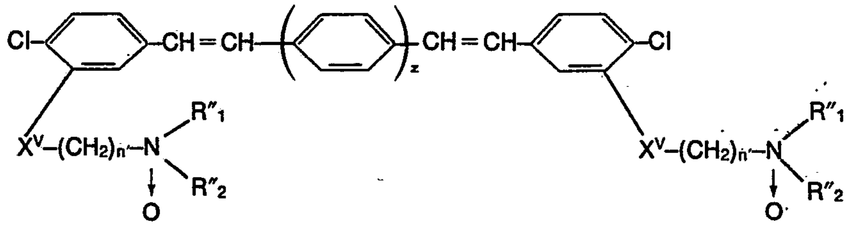

,

in which z is the integer 1 or 2, $X^V$ is an oxygen atom or a group of the formula $-SO_2N(R')$, R' being hydrogen or alkyl which has 1 to 4 carbon atoms and is unsubstituted or substituted by hydroxyl, cyano or halogen, n' is an integer between 1 and 3 and $R_1''$ and $R_2''$ independently of one another are alkyl or hydroxyalkyl having 1 to 4 carbon atoms.

5. An amine oxide compound according to claim 3, of the formula

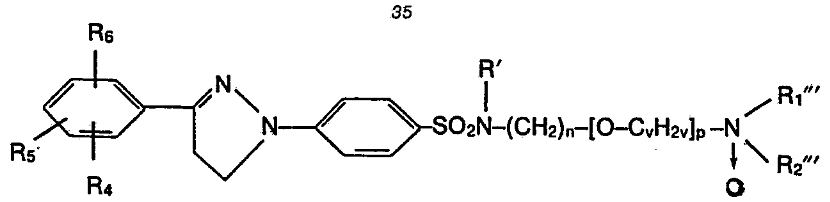

,

in which $R_4$ is hydrogen or halogen, $R_5$ and $R_6$ are each hydrogen, halogen, alkyl or alkoxy each having 1 to 4 carbon atoms, n and v are each an integer between 1 and 4, p is 0 or 1, R' is hydrogen or alkyl which has 1 to 4 carbon atoms and is unsubstituted or substituted by hydroxyl, cyano or halogen, $R_1'''$ and $R_2'''$ independently of one another are alkyl which has 1 to 8 carbon atoms and is unsubstituted or substituted by halogen, cyano, hydroxyl, alkoxy having 1 to 4 carbon atoms, phenyl, chlorophenyl, methoxyphenyl, methylphenyl or alkoxycarbonyl having 2 to 5 carbon atoms, or, if n is 2 or 3 and p is 0, R' and $R_1'''$ conjointly are an ethylene or methylene group, thereby forming a group of the formula

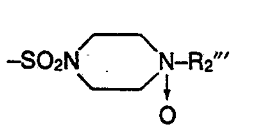

,

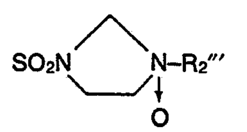

or

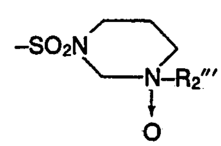

in particular, a compound of the formula

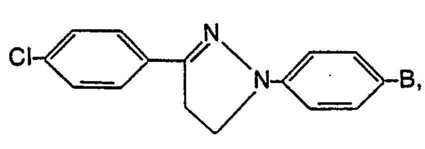

in which B is a group of the formula

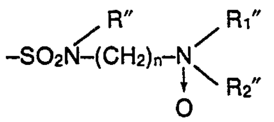
,
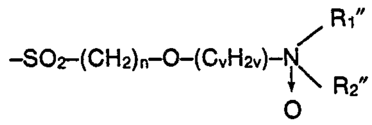
or
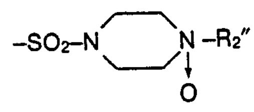

in which R″ is hydrogen or alkyl, hydroxyalkyl or cyanoalkyl, each having 1 to 4 carbon atoms in the alkyl moiety, $R_1''$ and $R_2''$ are each alkyl or hydroxyalkyl having 1 to 4 carbon atoms and n and v are each an integer between 1 and 4.

6. An amine oxide compound according to claim 3, of the formula

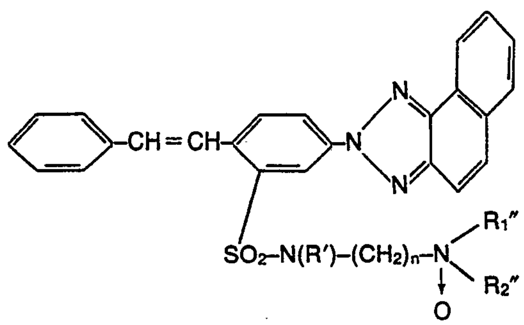
,

in which R′ is hydrogen or alkyl, hydroxyalkyl, cyanoalkyl or halogenoalkyl, each having 1 to 4 carbon atoms in the alkyl moiety, $R_1''$ and $R_2''$ are each alkyl or hydroxyalkyl having 1 to 4 carbon atoms and n is an integer between 1 and 4.

7. An amine oxide compound according to claim 3, of the formula

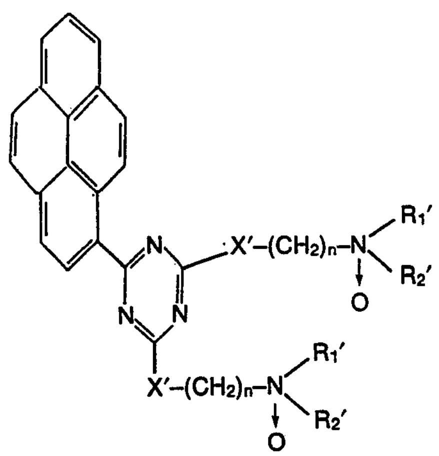

in which n is an integer between 1 and 4 and X′, $R_1'$ and $R_2'$ are as defined in claim 3, X′ preferably being oxygen and $R_1'$ and $R_2'$ preferably being, independently of one another, alkyl or hydroxyalkyl having 1 to 4 carbon atoms.

8. A process for the preparation of an amine oxide compound of the formula defined in claim 1, in which X is a direct bond between A and Y, an oxygen atom or a group of the formula $-SO_2-O-$, $-SO_2-$, $-COO-$, $-CON(R)-$ or $-SO_2N(R)-$, in which R is hydrogen or alkyl which is unsubstituted or substituted by halogen, hydroxyl, $C_2$–$C_5$carbalkoxy, $C_1$–$C_4$alkoxy, phenyl, chlorophenyl, methylphenyl, methoxyphenyl, carbamoyl, or sulfamoyl, which comprises oxidising a compound of the formula

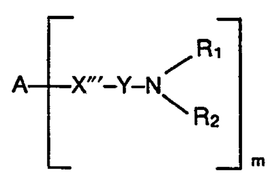
,

in which A, Y, $R_1$, $R_2$ and m are as defined in claim 1 and X‴ is a direct bond between A and Y, an oxygen atom or sulfur atom or a group of the formula $-SO-$, $-SO_2-$, $-SO_2-O-$, $-COO-$, $-CON(R)-$ or $-SO_2N(R)-$, in which R is as defined above, preferably by means of a per-compound which is employed in equivalent amount or in excess, in particular by means of performic acid, peracetic acid or hydrogen peroxide or a mixture of these per-compounds, and especially by means of hydrogen peroxide.

9. A process for the preparation of an amine oxide compound of the formula defined in claim 1, in which X is an oxygen atom or sulfur atom or a group of the formula $-SO_2-$ or $-COO-$, which comprises reacting one mol equivalent of a compound of the formula

$$A-[-X''-H]_m$$

with m mol equivalents of a compound of the formula

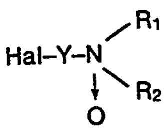

in which formulae A, m, Y, $R_1$ and $R_2$ are as defined in claim 1, Hal is chlorine or bromine and X″ is an oxygen atom or sulfur atom or a group of the formula $-SO_2-$ or $-COO-$.

10. A process for the preparation of an amine oxide compound of the formula defined in claim 1, in which X is a group of the formula $-COO-$ or $-SO_2-O-$, which comprises reacting one mol equivalent of a compound of the formula

$$A-[-SO_2Hal]_m \quad \text{or} \quad A-[-COHal]_m$$

with m mol equivalents of a compound of the formula

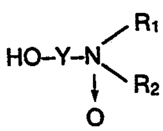

in which formulae A, m, Y, $R_1$ and $R_2$ are as defined in claim 1 and Hal is chlorine or bromine.

11. Use of an amine oxide compound of the formula given in claim 1 as a fluorescent brightener for high molecular weight organic materials, especially for polyacrylonitrile, polyamide and cellulose materials, preferably as a fluorescent brightener in a washing liquor.

12. A detergent containing one or more amine oxide compounds of the formula defined in claim 1.

13. A detergent according to claim 12, which contains 10 to 70% by weight of a non-ionic surfactant and 1 to 30% by weight of a cationic textile softener, the latter preferably being a quaternary derivative of ammonia or of imidazoline, having 2 long-chain aliphatic saturated or unsaturated radicals, or being a mixture of the two, which detergent may also contain a solvent to ensure its liquid form.

14. A detergent according to claim 13, which contains 1-methyl-1-oleyl-amidoethyl-2-oleyl-imidazolinium · $X^{\ominus}$, 1-methyl-1-talloylamido-ethyl-2-tallylimidazolinium · $X^{\ominus}$, ditallyl-dimethyl-ammonium · $X^{\ominus}$ or a compound of the formula

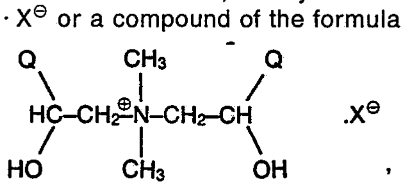

,

in which Q is $C_{14}-C_{16}$alkyl and $X^{\ominus}$ is a chloride, bromide, methylsulfate, ethylsulfate, methanesulfonate, ethanesulfonate or toluenesulfonate anion, as a cationic textile softener.

15. A textile treatment agent or laundry aftertreatment agent containing one or more amine oxide compounds of the formula defined in claim 1, and a cationic textile treatment agent or a cationic textile softener.

**Revendications**

1. Composés oxydes d'amine de formule

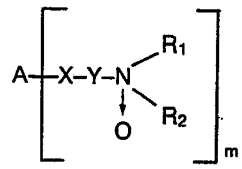

,

où

m représente le nombre 1 ou 2,

A représente le radical d'un système azurant du groupe des 2-furanyl-benzimidazole, 2-azolyl-benzimidazole, 2-stilbényl-benzoxazole, 2-stilbényl-benzimidazole, 1,2-bis-azolyléthylène, 2,5-bis-benzimidazolylfuranne, 4,4′-bis-azolylstilbène, 2-phényl-5-azolylthiophène, 1,3-diphénylpyrazoline, 4,4′-distyrylbiphényle, 4,4′-divinyl-stilbène, 1,4-distyrylbenzène, coumarine disubstituée en 3,7, naphtalimide, 2-stilbén-4-yl-naphtotriazole, 4,4′-bis-triazolyl- ou pyrazolyl-stilbène, triazolyl- ou pyrazolyl-stilbène, naphtotriazolyl-stilbène, triazinylpyrène ou bis-styryl-dibenzofuranne, ces radicaux étant non substitués ou substitués par halogène, alkyle, hydroxyalkyle, halogénalkyle, cyanoalkyle, alkoxyalkyle, phénylalkyle, carboxyalkyle, carbalkoxyalkyle, alcényle, cycloalkyle, alkoxy, alcényloxy, alkoxycarbonyle, aminocarbonyle, alkyl- ou dialkylaminocarbonyle, cyano, alkylsulfonyle, alkoxysufonyle, aminosulfonyle, hydroxy, carboxy, sulfo ou trifluorométhyle

X représente une liaison directe entre A et Y, un atome d'oxygène ou de soufre ou un groupe de formule $-SO_2-$, $-SO_2-O-$, $-COO-$, $-CON(R)-$ ou $-SO_2N(R)-$, dans lequel R représente un hydrogène ou un alkyle non substitué ou substitué par halogène, hydroxy, carbalkoxy en $C_{2-5}$, alkoxy en $C_{1-4}$, phényle, chlorophényle, méthylphényle, méthoxyphényle, carbamoyle ou sulfamoyle.

Y représente un groupe alkylène- ou alkylènoxyalkylène à chaîne droite ou ramifiée, non substitué ou substitué par halogène, hydroxy, carbalkoxy en $C_{2-5}$, alkoxy en $C_{1-4}$, phényle, chlorophényle, méthylphényle, méthoxyphényle, carbamoyle ou sulfamoyle.

$R_1$ et $R_2$ représentent indépendamment l'un de l'autre un cyclohexyle, un alkyle non substitué ou substitué par halogène, hydroxy, carbalkoxy en $C_{2-5}$, alkoxy en $C_{1-4}$, phényle, chlorophényle, méthylphényle, méthoxyphényle, carbamoyle ou sulfamoyle, un phényle, ou $R_1$ et $R_2$ représentent ensemble, avec l'atome d'azote auquel ils sont liés, un hétérocycle saturé ou non saturé à 5 ou 6 chaînons qui peut éventuellement contenir encore un ou deux autres hétéroatomes comme chaînons, un hétérocycle saturé pouvant éventuellement être substitué par 1 ou 2 groupes alkyle en $C_{1-4}$, et un hétérocycle insaturé pouvant être substitué par halogène, alkyle ou alkoxy; ou bien au cas où X représente un groupe de formule $-CON(R)-$ ou $-SO_2N(R)-$

et Y représente un éthylène ou un propylène, R et $R_1$ représentent ensemble le groupe éthylène ou méthylène, ce qui fait apparaître un hétérocycle saturé correspondant contenant 2 atomes d'azote.

2. Composés oxydes d'amine selon la revendication 1 où

X représente un atome d'oxygène ou de soufre ou un groupe de formule $-SO_2-$, $-CON(R)-$ ou $-SO_2N(R)-$ et Y représente un groupe alkylène à chaîne droite ou ramifiée comportant de 1 à 12 atomes de carbone,

R étant défini comme dans la revendication 1 et R et $R_1$ pouvant le cas échéant avoir ensemble la signification donnée dans la revendication 1.

3. Composés oxydes d'amine selon la revendication 1, de formule

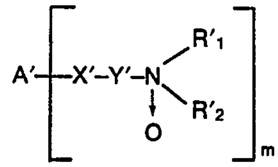

,

ou A′ représente un radical, non substitué ou substitué par halogène, alkyle, hydroxyalkyle, halogènalkyle, cyanoalkyle, alkoxyalkyle, phénylalkyle, carboxyalkyle, carbalkoxyalkyle, alcényle, cycloalkyle, alkoxy, alcényloxy, alkoxycarbonyl, aminocarbonyl, alkyl- ou dialkylaminocarbonyle, cyano, alkylsulfonyle, alkoxysulfonyle, aminosulfonyle, hydroxy, carboxy, sulfo ou trifluorométhyle, de formule

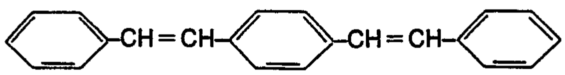

,

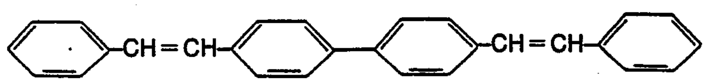

,

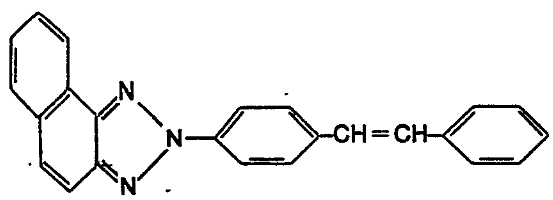

,

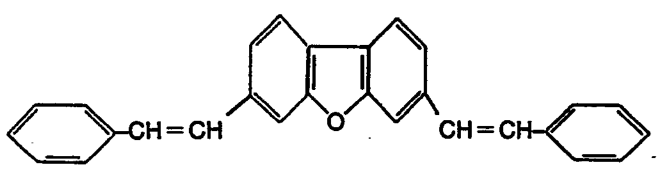

,

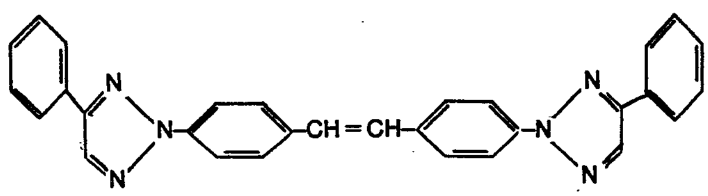

,

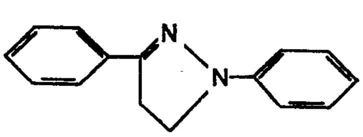

ou

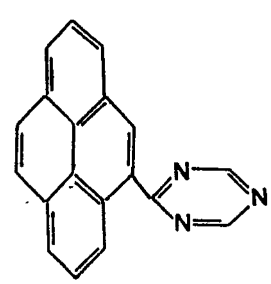

,

X′ représente un atome d'oxygène ou un groupe de formule $-SO_2N(R')-$ ou $-SO_2-$, dans lequel

R′ représente un hydrogène ou un alkyle non substitué ou substitué par hydroxy, cyano ou halogène, comportant de 1 à 4 atomes de carbone dans la partie alkyle,

Y′ représente un groupe alkylène ou alkylènoxyalkylène à chaîne droite ou ramifiée,

m représente le nombre 1 ou 2 et

$R_1'$ et $R_2'$ représentent indépendamment l'un de l'autre un alkyle, non substitué ou substitué par halogène, cyano, hydroxy, alkoxy et $C_{1-4}$, phényle, chlorophényle, méthoxyphényle, méthylphényle, ou alkoxycarbonyle comportant de 2 à 5 atomes de carbone, et comportant de 1 à 8 atomes de carbone dans la partie alkyle, ou $R_1'$ et $R_2'$ représentent ensemble, avec l'atome d'azote auquel ils sont liés, un hétérocycle saturé ou non saturé à 5 ou 6 chaînons qui peut encore contenir éventuellement comme chaînons un ou deux autres hétéroatomes, un hétérocycle éventuellement saturé pouvant être substitué par un ou deux groupes alkyle en $C_{1-4}$ et un hétérocycle non saturé pouvant être substitué par halogène, alkyle ou alkoxy; ou bien dans le cas où X′ représente un groupe de formule $-SO_2N(R')-$ et Y′ représente un éthylène ou un propylène, R′ et $R_1'$ représentent ensemble le groupe éthylène ou méthylène, ce qui fait apparaître un groupement de formule

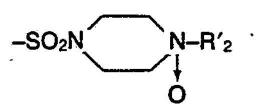
,
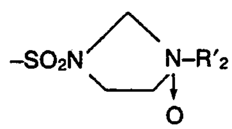
ou
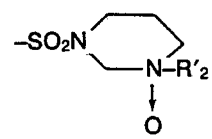

4. Composés oxydes d'amine selon la revendication 3 de formule

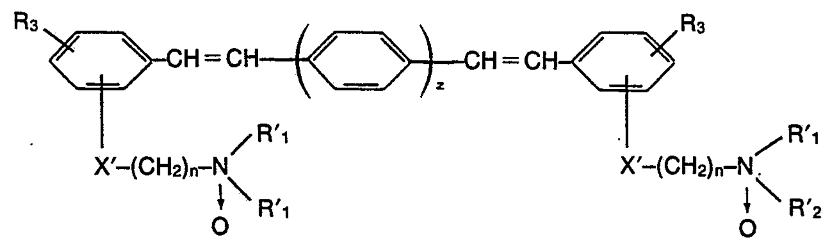

où Z représente le nombre 1 ou 2 et n représente un nombre entier compris entre 1 et 4, X′, $R_1'$ et $R_2'$ sont définis comme dans la revendication 3 et $R_3$ représente un halogène, alkyle comportant de 1 à 4 atomes de carbone, cycloalkyle comportant 5 ou 6 atomes de carbone, alkoxy en $C_{1-4}$, alkylsulfonyle en $C_{1-4}$, phénylsulfonyle, carbalkoxy en $C_{2-5}$, carbamoyle ou sulfamoyle, en particulier composés de formule

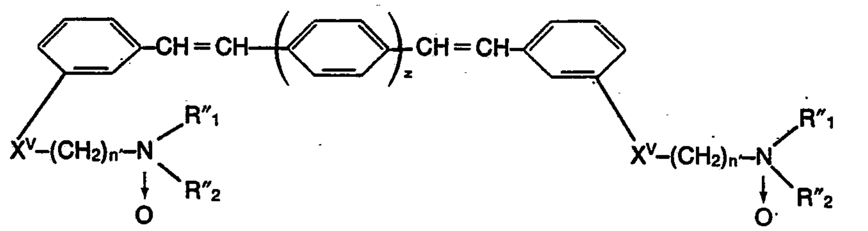

où

z représente le nombre 1 ou 2,

$X^v$ représente un atome d'oxygène ou un groupe de formule $-SO_2N(R')-$,

où R représente un hydrogène ou un alkyle, non substitué ou substitué par hydroxy, cyano ou halogène, et comportant de 1 à 4 atomes de carbone dans la partie alkyle,

n′ représente un nombre entier compris entre 1 et 3 et

$R_1''$ et $R_2''$ représentent indépendamment l'un de l'autre un alkyle ou hydroxyalkyle en $C_{1-4}$, ainsi que de formule

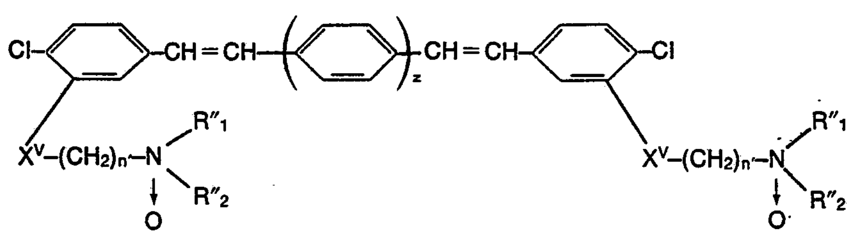

où

z représente le nombre 1 ou 2,

$X^v$ représente un atome d'oxygène ou un groupe de formule $-SO_2N(R')$

où R′ représente un hydrogène ou un alkyle, non substitué ou substitué par hydroxy, cyano ou halogène, et comportant de 1 à 4 atomes de carbone dans la partie alkyle,

n′ représente un nombre entier compris entre 1 et 3 et

$R_1''$ et $R_2''$ représentent indépendamment l'un de l'autre un alkyle ou hydroxyalkyle comportant de 1 à 4 atomes de carbone.

5. Composés oxydes d'amine selon la revendication 3 de formule

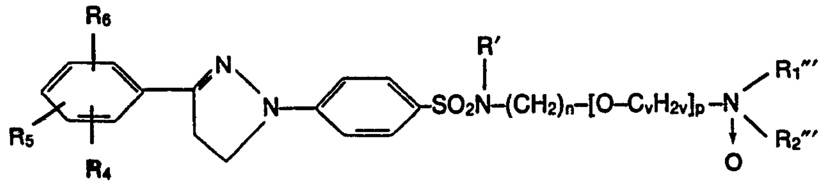

où $R_4$ représente un hydrogène ou un halogène, $R_5$ et $R_6$ représentent chacun un hydrogène, halogène, alkyle ou alkoxy comportant chacun de 1 à 4 atomes de carbone, n et v représentent chacun un nombre entier compris entre 1 et 4, p vaut 0 ou 1, R′ représente un hydrogène ou un alkyle non substitué ou substitué par hydroxy, cyano ou halogène, et comportant de 1 à 4 atomes de carbone dans la partie alkyle,

$R_1'''$ et $R_2'''$ reprèsentent indépendamment l'un de l'autre un alkyle, non substitué ou substitué par halogène, cyano, hydroxy, alkoxy en $C_{1-4}$, phényle, chlorophényle, méthoxyphényle, méthylphényle ou alkoxycarbonyle en $C_{2-5}$, et comportant de 1 à 8 atomes de carbone dans la partie alkyle, ou bien, dans le cas où n vaut 2 ou 3 et p vaut 0, R′ et $R_1'''$ représentent ensemble le groupe éthylène ou méthylène, ce qui fait apparaître un groupement de formule

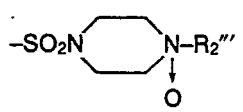 , 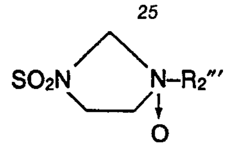 ou 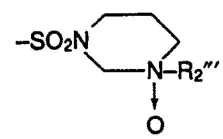

en particulier les composés de formule

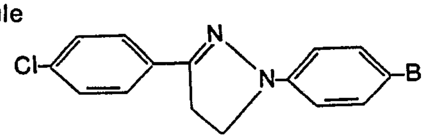 ,

où B représente un groupe de formule

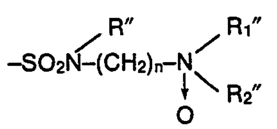 , 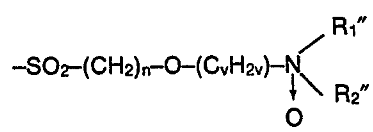 ou 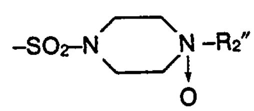

où R″ représente un hydrogène, alkyle, hydroxyalkyle ou cyanoalkyle avec à chaque fois de 1 à 4 atomes de carbone dans la partie alkyle, $R_1''$ et $R_2''$ représentent à chaque fois un alkyle ou un hydroalkyle comportant de 1 à 4 atomes de carbone et n et v représentent à chaque fois un nombre entier compris entre 1 et 4.

6. Composés oxydes d'amine selon la revendication 3 de formule

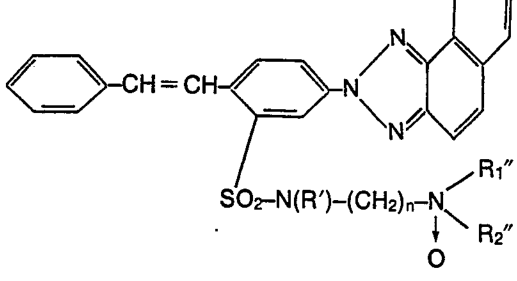 ,

où R′ représente un hydrogène, alkyle, hydroxyalkyle, cyanoalkyle ou halogénalkyle avec à chaque fois de 1 à 4 atomes de carbone dans la partie alkyle, $R_1''$ et $R_2''$ représentent chacun un alkyle ou un hydroxyalkyle comportant de 1 à 4 atomes de carbone et n représente un nombre entier compris entre 2 et 4.

7. Composés oxydes d'amine selon la revendication 3 de formule

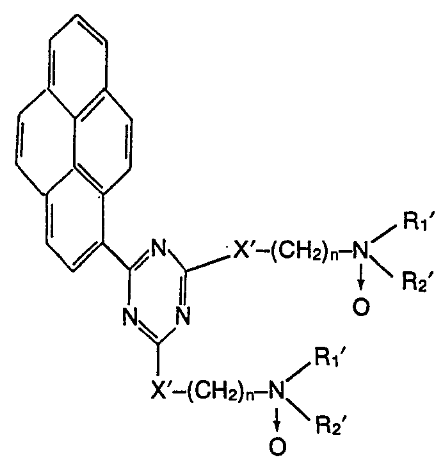

où n représente un nombre entier compris entre 1 et 4 et X', $R_1'$ et $R_2'$ sont définis comme dans la revendication 3, X' représentant de préférence un oxygène et $R_1'$ et $R_2'$ représentant de préférence indépendamment l'un de l'autre un alkyle ou un hydroxyalkyle en $C_{1-4}$.

8. Procédé de préparation de composés oxydes d'amine de formule définie dans la revendication 1, où X représente une liaison directe entre A et Y, un atome d'oxygène ou un groupe de formule $-SO_2-O-$, $-SO_2-$, $-COO-$, $-CON(R)-$ ou $-SO_2N(R)-$ où R représente un hydrogène ou un alkyle non substitué ou substitué par halogène, hydroxy, carbalkoxy en $C_{2-5}$, alkoxy en $C_{1-4}$, phényle, chlorophényle, méthylphényle, méthoxyphényle, carbamoyle ou sulfamoyle, caractérisé en ce qu'on oxyde un composé de formule

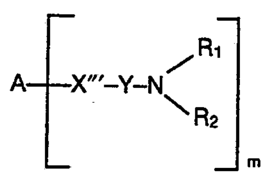,

où A, Y, $R_1$, $R_2$ et m sont définis comme dans la revendication 1 et X''' représente une liaison directe entre A et Y, un atome d'oxygène ou de soufre ou un groupe de formule $-SO-$, $-SO_2-$, $-SO_2-O-$, $-COO-$, $-CON(R)-$ ou $-SO_2N(R)-$ R étant tel que défini ci-dessus, de préférence avec un composé «per», que l'on utilise en quantité équivalente ou en excès, en particulier avec l'acide performique, l'acide peracétique ou le peroxyde d'hydrogène ou des mélanges de ces composés per, mais surtout avec le peroxyde d'hydrogène.

9. Procédé de préparation de composés oxydes d'amine de formule définie dans la revendication 1 où X représente un atome d'oxygène ou de soufre ou un groupe de formule $-SO_2-$ ou $-COO-$ caractérisé en ce qu'on fait réagir un équivalent molaire d'un composé de formule

$$A-[-X''-H]_m$$

avec m équivalents molaires d'un composé de formule

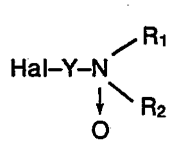

formules dans lesquelles A, m, Y, $R_1$ et $R_2$ sont définis comme dans la revendication 1, Hal représente un chlore ou un brome et X'' représente un atome d'oxygène ou de soufre ou un groupe de formule $-SO_2-$ ou $-COO-$.

10. Procédé de préparation de composés oxydes d'amine de formule définie dans la revendication 1, où X représente un groupe de formule $-COO-$ ou $-SO_2-O-$ caractérisé en ce qu'on fait réagir un équivalent molaire d'un composé de formule

$$A-[-SO_2Hal]_m \quad \text{ou} \quad A-[-COHal]_m$$

avec m équivalents molaires d'un composé de formule

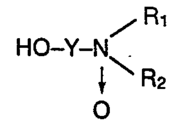

formules dans lesquelles A, m, Y, $R_1$ et $R_2$ sont tels que définis dans la revendication 1 et Hal représente un chlore ou un brome.

11. Application de composés oxydes d'amine de formule indiquée dans la revendication 1 comme azurants optiques pour les matières organiques à haut poids moléculaire, en particulier pour les matières faites de polyacrylonitrile, polyamide et cellulose, de préférence comme azurants optiques dans un bain de lavage.

12. Agent de lavage contenant un ou plusieurs composés oxydes d'amine de formule définie dans la revendication 1.

13. Agent de lavage selon la revendication 12, qui contient de 10 à 70% en poids d'un agent tensioactif non ionogène et de 1 à 30% en poids d'un assouplissant cationique pour textile, ce dernier étant de préférence un dérivé quaternaire de l'ammoniac ou de l'imidazoline avec 2 radicaux saturés ou non saturés aliphatiques à longue chaîne ou un mélange de deux, cet agent de lavage contenant éventuellement encore un solvant qui donne la forme liquide.

14. Agent de lavage selon la revendication 13, qui contient, comme assouplissant cationique pour textile, un 1-méthyl-1-oléylamidoéthyl-2-oléyl-imidazolinium · $X^\ominus$, 1-méthyl-1-talloylamidoéthyl-2-tallyl-imidazolinium · $X^\ominus$, di-tallyl-diméthyl-ammonium · $X^\ominus$ ou un composé de formule

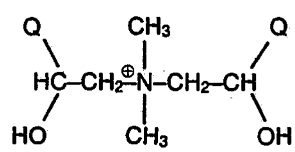 . $X^\ominus$ ,

où Q représente un alkyle en $C_{14-16}$ et $X^\ominus$ représente un anion chlorure, bromure, méthylsulfate, éthylsulfate, méthane-, éthane- ou toluène sulfonate.

15. Agent de traitement des textiles ou agent de post-traitement du linge contenant un ou plusieurs composés oxydes d'amine de formule définie dans la revendication 1 et un agent cationique de traitement des textiles ou un assouplissant cationique pour textile.